# EUROPEAN PATENT SPECIFICATION

(11) **EP 3 328 426 B1**
(45) Date of publication and mention of the grant of the patent: **07.04.2021**
(21) Application number: 16753597.0
(22) Date of filing: 27.07.2016
(51) Int. Cl.: A61K 45/06, A61K 31/337, A61K 31/517, A61K 33/24, A61P 35/00, A61K 31/7068, A61K 33/243, C12Q 1/6886, G01N 33/574, A61K 39/395, C07K 16/32

(54) **TREATMENT OF PATIENTS DIAGNOSED WITH PANCREATIC DUCTAL ADENOCARCINOMA USING USING MONOCLONAL ANTIBODIES AGAINST THE EPIDERMAL GROWTH FACTOR RECEPTOR (EGFR)**
BEHANDLUNG VON PATIENTEN MIT DIAGNOSTIZIERTEM DUKTALEN ADENOKARZINOM DES PANKREAS MIT MONOKLONALEN ANTIKÖRPERN GEGEN DEN EPIDERMALEN WACHSTUMSFAKTOR REZEPTOR (EGFR)
TRAITEMENT DES PATIENTS DIAGNOSTIQUÉS AVEC UN ADÉNOCARCINOME CANALAIRE PAR DES ANTICORPS MONOCLONAUX CONTRE LE RECEPTEUR DU FACTEUR DE CROISSANCE ÉPIDERMIQUE

(30) Priority: 27.07.2015 EP 15178489
(43) Date of publication of application: 06.06.2018
(73) Proprietor: InnoCIMAb Pte Ltd, Singapore 659065 (SG); Oncoscience AG, 22880 Wedel (DE)
(72) Inventor: STRUMBERG, Dirk, 44621 Herne (DE); SCHULTHEIS, Beate, Herne (DE); EBERT, Matthias, P., Munich (DE); SIVEKE, Jens, Munich (DE); KERKHOFF, Andrea, Muenster (DE); HOFHEINZ, Ralf, Mannheim (DE); BEHRINGER, Dirk, M., Bochum (DE); SCHMIDT, Wolfgang, E., Bochum (DE); GOKER, Erdem, Izmir (TR); DE DOSSO, Sara, Bellinzona (CH); KNEBA, Michael, Kiel (DE); YALCIN, Suayib, Ankara (TR); OVERKAMP, Friedrich, Recklinghausen (DE); SCHLEGEL, Frank, Eschweiler (DE); DOMMACH, Markus, Duesseldorf (DE); ROHRBERG, Robert, Halle (DE); STEINMETZ, Tilman, Cologne (DE); BACH, Ferdinand, 22880 Wedel (DE); REUTER, Dirk, 22880 Wedel (DE); ILYAS, Rikrik, Asiyah, Singapore 658065 (SG); IZNAGA ESCOBAR, Normando, E., Singapore 658065 (SG)
(74) Representative: Schiweck Weinzierl Koch Patentanwälte Partnerschaft mbB
(86) International application number: PCT/EP2016/067892
(87) International publication number: WO 2017/017133

(56) References cited:
- EP-A1- 2 070 547
- WO-A1-2013/138371
- WO-A1-2014/095766
- Sideways: "EGFR inhibition is effective against KRAS-wild-type disease | Nature Reviews Clinical Oncology", Nature reviews clinical oncology, 25 July 2017 (2017-07-25), XP55536776, Retrieved from the Internet: URL:https://www.nature.com/articles/nrclin onc.2017.119 [retrieved on 2018-12-19]
- B. SCHULTHEIS ET AL: "Gemcitabine combined with the monoclonal antibody nimotuzumab is an active first-line regimen in KRAS wildtype patients with locally advanced or metastatic pancreatic cancer: a multicenter, randomized phase IIb study", ANNALS OF ONCOLOGY., vol. 28, no. 10, 6 July 2017 (2017-07-06), pages 2429-2435, XP055652203, NL ISSN: 0923-7534, DOI: 10.1093/annonc/mdx343

### SEQUENCE LISTING

<110> InnoCIMAb Pte Ltd & Oncoscience AG
<120> TREATMENT OF PATIENTS DIAGNOSED WITH PANCREATIC DUCTAL ADENOCARCINOMA
<130> INN15364PCT
<160> 23
<170> PatentIn version 3.5
<210> 1
   <211> 16
   <212> PRT
   <213> Artificial Sequence
<220>
   <223> synthetic peptide
<400> 1
   Arg Ser Ser Gln Asn Ile Val His Ser Asn Gly Asn Thr Tyr Leu Asp
   1               5                   10                  15
<210> 2
   <211> 7
   <212> PRT
   <213> Artificial Sequence
<220>
   <223> synthetic peptide
<400> 2
   Lys Val Ser Asn Arg Phe Ser
   1               5
<210> 3
   <211> 9
   <212> PRT
   <213> Artificial Sequence
<220>
   <223> synthetic peptide
<400> 3
   Phe Gln Tyr Ser His Val Pro Trp Thr
   1               5
<210> 4
   <211> 5
   <212> PRT
   <213> Artificial Sequence
<220>
   <223> synthetic peptide
<400> 4
   Asn Tyr Tyr Ile Tyr
   1               5
<210> 5
   <211> 17
   <212> PRT
   <213> Artificial Sequence
<220>
   <223> synthetic peptide
<400> 5
   Gly Ile Asn Pro Thr Ser Gly Gly Ser Asn Phe Asn Glu Lys Phe Lys
   1               5                   10                  15
   Thr
<210> 6
   <211> 14
   <212> PRT
   <213> Artificial Sequence
<220>
   <223> synthetic peptide
<400> 6
   Gln Gly Leu Trp Phe Asp Ser Asp Gly Arg Gly Phe Asp Phe
   1               5                   10
<210> 7
   <211> 189
   <212> PRT
   <213> Homo sapiens
<400> 7
<210> 8
   <211> 189
   <212> PRT
   <213> Homo sapiens
<400> 8
<210> 9
   <211> 189
   <212> PRT
   <213> Homo sapiens
<400> 9
   Met Thr Glu Tyr Lys Leu Val Val Val Gly Ala Gly Gly Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Ser Lys Ser Phe Ala Asp Ile Asn Leu Tyr
                   85                  90                  95
   Arg Glu Gln Ile Lys Arg Val Lys Asp Ser Asp Asp Val Pro Met Val
               100                 105                 110
   Leu Val Gly Asn Lys Cys Asp Leu Pro Thr Arg Thr Val Asp Thr Lys
           115                 120                 125
   Gln Ala His Glu Leu Ala Lys Ser Tyr Gly Ile Pro Phe Ile Glu Thr
       130                 135                 140
   Ser Ala Lys Thr Arg Gln Gly Val Glu Asp Ala Phe Tyr Thr Leu Val
   145                 150                 155                 160
   Arg Glu Ile Arg Gln Tyr Arg Met Lys Lys Leu Asn Ser Ser Asp Asp
                   165                 170                 175
   Gly Thr Gln Gly Cys Met Gly Leu Pro Cys Val Val Met
               180                 185
<210> 10
   <211> 114
   <212> PRT
   <213> Artificial Sequence
<220>
   <223> synthetic peptide
<400> 10
   Asp Val Leu Met Thr Gln Ile Pro Leu Ser Leu Pro Val Ser Leu Gly
   1 5 10 15
   Asp Gln Ala Ser Ile Ser Cys Arg Ser Ser Gln Asn Ile Val His Ser
   20 25 30
   Asn Gly Asn Thr Tyr Leu Asp Trp Tyr Leu Gln Lys Pro Gly Gln Ser
   35 40 45
   Pro Asn Leu Leu Ile Tyr Lys Val Ser Asn Arg Phe Ser Gly Val Pro
   50 55 60
   Asp Arg Phe Arg Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Tyr
   85 90 95
   Ser His Val Pro Trp Thr Phe Gly Gly Gly Thr Lys Leu Glu Ile Lys
   100 105 110
   Arg Ala
<210> 11
   <211> 123
   <212> PRT
   <213> Artificial Sequence
<220>
   <223> synthetic peptide
<400> 11
   Gln Val Gln Leu Gln Gln Pro Gly Ala Glu Leu Val Lys Pro Gly Ala
   1 5 10 15
   Ser Val Lys Leu Ser Cys Lys Ala Ser Gly Tyr Thr Phe Thr Asn Tyr
   20 25 30
   Tyr Ile Tyr Trp Val Lys Gln Arg Pro Gly Gln Gly Leu Glu Trp Ile
   35 40 45
   Gly Gly Ile Asn Pro Thr Ser Gly Gly Ser Asn Phe Asn Glu Lys Phe
   50 55 60
   Lys Thr Lys Ala Thr Leu Thr Val Asp Glu Ser Ser Thr Thr Ala Tyr
   65 70 75 80
   Met Gln Leu Ser Ser Leu Thr Ser Glu Asp Ser Ala Val Tyr Tyr Cys
   85 90 95
   Thr Arg Gln Gly Leu Trp Phe Asp Ser Asp Gly Arg Gly Phe Asp Phe
   100 105 110
   Trp Gly Gln Gly Thr Thr Leu Thr Val Ser Ser
   115 120
<210> 12
   <211> 114
   <212> PRT
   <213> Artificial Sequence
<220>
   <223> synthetic peptide
<400> 12
   Asp Ile Gln Met Thr Gln Ser Pro Ser Ser Leu Ser Ala Ser Val Gly
   1 5 10 15
   Asp Arg Val Thr Ile Thr Cys Arg Ser Ser Gln Asn Ile Val His Ser
   20 25 30
   Asn Gly Asn Thr Tyr Leu Asp Trp Tyr Gln Gln Thr Pro Gly Lys Ala
   35 40 45
   Pro Lys Leu Leu Ile Tyr Lys Val Ser Asn Arg Phe Ser Gly Val Pro
   50 55 60
   Ser Arg Phe Ser Gly Ser Gly Ser Gly Thr Asp Phe Thr Phe Thr Ile
   65 70 75 80
   Ser Ser Leu Gln Pro Glu Asp Ile Ala Thr Tyr Tyr Cys Phe Gln Tyr
   85 90 95
   Ser His Val Pro Trp Thr Phe Gly Gln Gly Thr Lys Leu Gln Ile Thr
   100 105 110
   Arg Glu
<210> 13
   <211> 123
   <212> PRT
   <213> Artificial Sequence
<220>
   <223> synthetic peptide
<220>
   <221> misc_feature
   <222> (67)..(67)
   <223> Xaa can be Arginine or Lysine
<220>
   <221> misc_feature
   <222> (68)..(68)
   <223> Xaa can be Valine or Alanine
<220>
   <221> misc_feature
   <222> (76)..(76)
   <223> Xaa can be Threonine or Serine
<220>
   <221> misc_feature
   <222> (77)..(77)
   <223> Xaa can be Asparagine or Threonine
<220>
   <221> misc_feature
   <222> (97)..(97)
   <223> Xaa can be Alanine or Threonine
<400> 13
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Ile
           35                  40                  45
   Gly Gly Ile Asn Pro Thr Ser Gly Gly Ser Asn Phe Asn Glu Lys Phe
       50                  55                  60
   Lys Thr Xaa Xaa Thr Ile Thr Val Asp Glu Ser Xaa Xaa Thr Ala Tyr
   65                  70                  75                      80
   Met Glu Leu Ser Ser Leu Arg Ser Glu Asp Thr Ala Phe Tyr Phe Cys
                   85                  90                  95
   Xaa Arg Gln Gly Leu Trp Phe Asp Ser Asp Gly Arg Gly Phe Asp Phe
               100                 105                 110
   Trp Gly Gln Gly Ser Thr Val Thr Val Ser Ser
           115                 120
<210> 14
   <211> 114
   <212> PRT
   <213> Artificial Sequence
<220>
   <223> synthetic peptide
<400> 14
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Asn Ile Val His Ser
               20                  25                  30
   Asn Gly Asn Thr Tyr Leu Asp Trp Tyr Gln Gln Thr Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Phe Thr Ile
   65                  70                  75                  80
   Ser Ser Leu Gln Pro Glu Asp Ile Ala Thr Tyr Tyr Cys Phe Gln Tyr
                   85                  90                  95
   Ser His Val Pro Trp Thr Phe Gly Gln Gly Thr Lys Leu Gln Ile Thr
               100                 105                 110
   Arg Glu
<210> 15
   <211> 123
   <212> PRT
   <213> Artificial Sequence
<220>
   <223> synthetic peptide
<400> 15
   Gln Val Gln Leu Gln Gln Ser Gly Ala Glu Val Lys Lys Pro Gly Ser
   1 5 10 15
   Ser Val Lys Val Ser Cys Lys Ala Ser Gly Tyr Thr Phe Thr Asn Tyr
   20 25 30
   Tyr Ile Tyr Trp Val Arg Gln Ala Pro Gly Gln Gly Leu Glu Trp Ile
   35 40 45
   Gly Gly Ile Asn Pro Thr Ser Gly Gly Ser Asn Phe Asn Glu Lys Phe
   50 55 60
   Lys Thr Arg Val Thr Ile Thr Val Asp Glu Ser Ser Thr Thr Ala Tyr
   65 70 75 80
   Met Glu Leu Ser Ser Leu Arg Ser Glu Asp Thr Ala Phe Tyr Phe Cys
   85 90 95
   Thr Arg Gln Gly Leu Trp Phe Asp Ser Asp Gly Arg Gly Phe Asp Phe
   100 105 110
   Trp Gly Gln Gly Ser Thr Val Thr Val Ser Ser
   115 120
<210> 16
   <211> 114
   <212> PRT
   <213> Artificial Sequence
<220>
   <223> synthetic peptide
<400> 16
   Asp Ile Gln Met Thr Gln Ser Pro Ser Ser Leu Ser Ala Ser Val Gly
   1 5 10 15
   Asp Arg Val Thr Ile Thr Cys Arg Ser Ser Gln Asn Ile Val His Ser
   20 25 30
   Asn Gly Asn Thr Tyr Leu Asp Trp Tyr Gln Gln Thr Pro Gly Lys Ala
   35 40 45
   Pro Lys Leu Leu Ile Tyr Lys Val Ser Asn Arg Phe Ser Gly Val Pro
   50 55 60
   Ser Arg Phe Ser Gly Ser Gly Ser Gly Thr Asp Phe Thr Phe Thr Ile
   65 70 75 80
   Ser Ser Leu Gln Pro Glu Asp Ile Ala Thr Tyr Tyr Cys Phe Gln Tyr
   85 90 95
   Ser His Val Pro Trp Thr Phe Gly Gln Gly Thr Lys Leu Gln Ile Thr
   100 105 110
   Arg Glu
<210> 17
   <211> 123
   <212> PRT
   <213> Artificial Sequence
<220>
   <223> synthetic peptide
<400> 17
   Gln Val Gln Leu Gln Gln Ser Gly Ala Glu Val Lys Lys Pro Gly Ser
   1 5 10 15
   Ser Val Lys Val Ser Cys Lys Ala Ser Gly Tyr Thr Phe Thr Asn Tyr
   20 25 30
   Tyr Ile Tyr Trp Val Arg Gln Ala Pro Gly Gln Gly Leu Glu Trp Ile
   35 40 45
   Gly Gly Ile Asn Pro Thr Ser Gly Gly Ser Asn Phe Asn Glu Lys Phe
   50 55 60
   Lys Thr Lys Ala Thr Ile Thr Val Asp Glu Ser Ser Thr Thr Ala Tyr
   65 70 75 80
   Met Glu Leu Ser Ser Leu Arg Ser Glu Asp Thr Ala Phe Tyr Phe Cys
   85 90 95
   Thr Arg Gln Gly Leu Trp Phe Asp Ser Asp Gly Arg Gly Phe Asp Phe
   100 105 110
   Trp Gly Gln Gly Ser Thr Val Thr Val Ser Ser
   115 120
<210> 18
   <211> 114
   <212> PRT
   <213> Artificial Sequence
<220>
   <223> synthetic peptide
<400> 18
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Asn Ile Val His Ser
               20                  25                  30
   Asn Gly Asn Thr Tyr Leu Asp Trp Tyr Gln Gln Thr Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Phe Thr Ile
   65                  70                  75                  80
   Ser Ser Leu Gln Pro Glu Asp Ile Ala Thr Tyr Tyr Cys Phe Gln Tyr
                   85                  90                  95
   Ser His Val Pro Trp Thr Phe Gly Gln Gly Thr Lys Leu Gln Ile Thr
               100                 105                 110
   Arg Glu
<210> 19
   <211> 123
   <212> PRT
   <213> Artificial Sequence
<220>
   <223> synthetic peptide
<400> 19
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Ile
           35                  40                  45
   Gly Gly Ile Asn Pro Thr Ser Gly Gly Ser Asn Phe Asn Glu Lys Phe
       50                  55                  60
   Lys Thr Arg Val Thr Ile Thr Val Asp Glu Ser Ser Thr Thr Ala Tyr
   65                  70                  75                  80
   Met Glu Leu Ser Ser Leu Arg Ser Glu Asp Thr Ala Phe Tyr Phe Cys
                   85                  90                  95
   Ala Arg Gln Gly Leu Trp Phe Asp Ser Asp Gly Arg Gly Phe Asp Phe
               100                 105                 110
   Trp Gly Gln Gly Ser Thr Val Thr Val Ser Ser
           115                 120
<210> 20
   <211> 114
   <212> PRT
   <213> Artificial Sequence
<220>
   <223> synthetic peptide
<400> 20
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Asn Ile Val His Ser
               20                  25                  30
   Asn Gly Asn Thr Tyr Leu Asp Trp Tyr Gln Gln Thr Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Phe Thr Ile
   65                  70                  75                  80
   Ser Ser Leu Gln Pro Glu Asp Ile Ala Thr Tyr Tyr Cys Phe Gln Tyr
                   85                  90                  95
   Ser His Val Pro Trp Thr Phe Gly Gln Gly Thr Lys Leu Gln Ile Thr
               100                 105                 110
   Arg Glu
<210> 21
   <211> 123
   <212> PRT
   <213> Artificial Sequence
<220>
   <223> synthetic peptide
<400> 21
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Ile
           35                  40                  45
   Gly Gly Ile Asn Pro Thr Ser Gly Gly Ser Asn Phe Asn Glu Lys Phe
       50                  55                  60
   Lys Thr Arg Val Thr Ile Thr Val Asp Glu Ser Thr Asn Thr Ala Tyr
   65                  70                  75                  80
   Met Glu Leu Ser Ser Leu Arg Ser Glu Asp Thr Ala Phe Tyr Phe Cys
                   85                  90                  95
   Thr Arg Gln Gly Leu Trp Phe Asp Ser Asp Gly Arg Gly Phe Asp Phe
               100                 105                 110
   Trp Gly Gln Gly Ser Thr Val Thr Val Ser Ser
           115                 120
<210> 22
   <211> 114
   <212> PRT
   <213> Artificial Sequence
<220>
   <223> synthetic peptide
<400> 22
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Asn Ile Val His Ser
               20                  25                  30
   Asn Gly Asn Thr Tyr Leu Asp Trp Tyr Gln Gln Thr Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Phe Thr Ile
   65                  70                  75                  80
   Ser Ser Leu Gln Pro Glu Asp Ile Ala Thr Tyr Tyr Cys Phe Gln Tyr
                   85                  90                  95
   Ser His Val Pro Trp Thr Phe Gly Gln Gly Thr Lys Leu Gln Ile Thr
               100                 105                 110
   Arg Glu
<210> 23
   <211> 123
   <212> PRT
   <213> Artificial Sequence
<220>
   <223> synthetic peptide
<400> 23
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Ile
           35                  40                  45
   Gly Gly Ile Asn Pro Thr Ser Gly Gly Ser Asn Phe Asn Glu Lys Phe
       50                  55                  60
   Lys Thr Lys Ala Thr Ile Thr Val Asp Glu Ser Thr Asn Thr Ala Tyr
   65                  70                  75                  80
   Met Glu Leu Ser Ser Leu Arg Ser Glu Asp Thr Ala Phe Tyr Phe Cys
                   85                  90                  95
   Thr Arg Gln Gly Leu Trp Phe Asp Ser Asp Gly Arg Gly Phe Asp Phe
               100                 105                 110
   Trp Gly Gln Gly Ser Thr Val Thr Val Ser Ser
           115                 120

## Description

### BACKGROUND

Pancreatic cancer is the fourth leading cause of cancer death with approximately 48,960 new cases (3 % of all new cancer cases) and about 40,560 (6.9 % of all cancer cases) of deaths estimated in the United States in 2015. The majority of cases (about 99%) occur in the exocrine component of the pancreas. There are several sub-types of exocrine pancreatic cancers, but their diagnosis and treatment have much in common. The small minority of cancers that arise in the endocrine tissue of the pancreas have different clinical characteristics. Both groups affect mainly (but not exclusively) in people over 40, and are slightly more common in men, but some rare sub-types mainly occur in women or children.

Pancreatic cancer is aggressive with few symptoms until the cancer is advanced. Symptoms may include abdominal pain, weight loss, diarrhea, and jaundice. Pancreatic cancer has a very poor prognosis, as they it is normally diagnosed at a late stage when the cancer is already locally advanced or has spread to other parts of the body.

Pancreatic ductal adenocarcinoma (PDAC) is the predominant form of pancreatic cancer, representing about 85% of all pancreatic cancers. It is almost universally fatal. The annual number of deaths equals the number of newly diagnosed cases, despite intense treatment. The overall 5-year survival rate of only 5% has remained unchanged over the last 30 years, despite tremendous efforts in preclinical and clinical science.

Ras proteins are proto-oncogenes that are frequently mutated in human cancers. They are encoded by three ubiquitously expressed genes: HRAS, KRAS and NRAS. Recent exome sequencing established that KRAS is the most frequently mutated gene in PDAC (95%). The three human RAS genes HRAS, NRAS, and KRAS encode four highly homologous (83-90% sequence identity) 21-kDa small GTPases which cycle between a GTP-bound 'on' state and a GDP-bound 'off' state regulating pathways responsible for proliferation and cell survival (Bryant et al. Trends Biochem Sci. 2014 Feb;39(2):91-100.). Ras proteins are normally tightly regulated by guanine nucleotide exchange factors (GEFs) promoting GDP dissociation and GTP binding and GTPase-activating proteins (GAPs) that stimulate the intrinsic GTPase activity of Ras to switch off signalling. Aberrant Ras function is associated with hyper-proliferative developmental disorders and cancer. Activating mutations of KRAS found in human PDAC (point mutations at codon G12 (98% of all KRAS mutations in PDAC), G13 and Q61) impairs KRAS inactivation, thereby leading to constitutive activation of KRAS and persistent stimulation of downstream signalling pathways that drive many of the hallmarks of cancer, sustained proliferation, metabolic reprogramming, anti-apoptosis, remodelling of the tumour microenvironment, evasion of the immune response, cell migration and metastasis (Eser et al. Br J Cancer. 2014 Aug 26;111(5):817-22). Importantly, KRAS mutation is also an early and initiating event because it has been shown that over 90% of low-grade pancreatic intraepithelial neoplasia (PanIN) lesions harbor oncogenic KRAS mutations (Bryant et al., loc. cit.). Activating mutations of HRAS have been also found on human PDAC (point mutations at codon G12D, 49.7 % (1312 mutations out of 2639 tumor samples) and at codon G12V, 30.8% (812 mutations out of 2639 tumor samples). The catalogue of somatic mutations in cancer (COSMIC) who represents the most comprehensive database on human tumor mutations currently available (Forbes S.A. et al. Nucleic Acids Res. 2011, 39 (Database issue): D945-950) despite relative low sample size reveled an strong trends that KRAS is the most frequently mutated isoform in pancreatic cancer with a 61 % of mutations (3127 mutations in 5169 tumor samples), followed by NRAS with a 2 % of mutations (5 mutations in 248 tumor samples) and HRAS with 0 % (no mutations found in 221 tumor samples).

To date, surgery is the only cure for pancreatic cancer and in particular PDAC. However, surgery is only indicated in around 20% of new cases. Current standard of care for PDAC patients also includes gemcitabine, a nucleoside analogue that, without additional surgery, only prolongs survival for few weeks. Gemcitabine can be also used in combination therapies, for example, WO2013/138371 discloses methods for treating pancreatic cancer using combination therapies comprising an anti-Erbb3 antibody in combination with gemcitabine.The FOLFIRINOX chemotherapy regimen using four drugs was found more effective than gemcitabine, but has substantial side effects. This is also true of protein-bound paclitaxel (nab-paclitaxel) (von Hoff et al. N Engl J Med. 2013 Oct 31;369(18):1691-703).

Due to the considerable experimental evidence for the role of mutant KRAS and HRAS as a drivers of cancer development and growth in a large subpopulation of PDAC patients, there have been extensive efforts to develop direct KRAS or HRAS inhibitors. However, all clinical attempts to directly interfere with KRAS or HRAS activity have failed, and KRAS or HRAS is still widely considered undruggable (Eser et al., loc. cit.).

The role of other signaling pathways in PanIN progression and PDAC maintenance is the subject of ongoing research. Moore et al. J Clin Oncol. 2007 May 20;25(15): 1960-6 reported that treatment with the EGFR inhibitor Erlotinib has shown benefit in about 10% of PDAC patients, but failed to identify the underlying characteristics that would allow to distinguish responsive from non-responsive patients.

However, to date, these strategies have not transitioned any effective PDAC treatment into the clinic. Comprehensive genetic analysis has revealed that PDAC is an extremely complex and heterogeneous disease with at least three different subtypes, which exhibit distinct differences in many important aspects of cancer cell biology including cell morphology, gene expression, and in vitro response to therapy (Bryant et al., loc. cit). There is unquestionably an urgent need to provide new PDAC therapeutics taking into account the differences in the PDAC subtypes, and in particular in specifically targeting PDAC subpopulations. Due to the abundance of PDAC characterized by expression of mutant KRAS; or HRAS research and the development of novel PDAC therapeutics has predominantly focused on the same, while there is still an urgent need for customized treatment of other PDAC subtypes, in particular in such expressing wild-type KRAS, HRAS or NRAS.

It was the aim of the present invention to address the needs in the prior art as set out herein.

### SUMMARY

In a first aspect, the present invention relates to a monoclonal antibody, or an antigen binding fragment or peptide thereof which specifically binds epidermal growth factor receptor (EGF-R) for use in a method of treating a population of patients having been diagnosed with pancreatic ductal adenocarcinoma (PDAC), wherein the pancreatic ductal adenocarcinoma expresses the wild-type protein of at least one member of the RAS gene family selected from the KRAS wild-type, the HRAS wild-type or the NRAS wild-type, the method comprising administering to the patient a monoclonal antibody or an antigen binding fragment or peptide thereof which specifically binds epidermal growth factor receptor (EGF-R), wherein the antibody is a chimeric or a humanized monoclonal antibody which contains the following complementarity determining regions (CDRs) :
CDR1 of the light chain: RSSQNIVHSNGNTYLD (SEQ ID NO: 1),
CDR2 of the light chain: KVSNRFS (SEQ ID NO: 2),
CDR3 of the light chain: FQYSHVPWT (SEQ ID NO: 3),
CDR1 of the heavy chain: NYYIY (SEQ ID NO: 4),
CDR2 of the heavy chain: GINPTSGGSNFNEKFKT (SEQ ID NO: 5),
CDR3 of the heavy chain: QGLWFDSDGRGFDF (SEQ ID NO: 6).

It is envisaged that in one embodiment the monoclonal antibody or antigen-binding fragment thereof is produced by the cell line h-R3 deposited as ECACC 951110101. In particular, the monoclonal antibody may be nimotuzumab (Ramakrishnan et al. MAbs. 2009 Jan-Feb; 1(1): 41-48), the amino acid sequence of which is also described in European Patent EP 0 712 863 B1.

The population of patients may be selected by determining the presence or absence of mutant and/or wild-type KRAS, mutant or wild-type HRAS, mutant or wild-type NRAS in a PDAC tumor sample of each patient. Patients in whose tumor samples mutant KRAS, HRAS or NRAS is detected and/or wild-type KRAS, HRAS or NRAS is not detected are not selected for treatment, whereas patients in whose wild-type KRAS, HRAS or NRAS is detected and/or mutant KRAS, HRAS or NRAS is not detected are selected for treatment.

In a further aspect, a pharmaceutical composition comprising a monoclonal antibody or an antigen binding fragment or peptide thereof as defined herein is provided, said pharmaceutical composition being for use in treating a population of patients having been diagnosed with pancreatic ductal adenocarcinoma (PDAC), wherein the pancreatic ductal adenocarcinoma expresses the wild-type protein of at least one member of the RAS gene family selected from consisting of the KRAS wild-type, the NRAS wild-type and the HRAS wild-type.

In a further aspect, the present invention relates to a monoclonal antibody, or an antigen binding fragment or peptide thereof which specifically binds epidermal growth factor receptor (EGF-R) for use in treating a population of patients having been diagnosed with pancreatic ductal adenocarcinoma (PDAC), wherein the pancreatic ductal adenocarcinoma expresses the wild-type protein of at least one member of the RAS gene family selected from the group consisting of the KRAS wild-type, the NRAS wild-type and the HRAS wild-type and wherein the antibody is a chimeric or a humanized antibody which contains the following complementarity determining regions (CDRs) :
CDR1 of the light chain: RSSQNIVHSNGNTYLD (SEQ ID NO: 1),
CDR2 of the light chain: KVSNRFS (SEQ ID NO: 2),
CDR3 of the light chain: FQYSHVPWT (SEQ ID NO: 3),
CDR1 of the heavy chain: NYYIY (SEQ ID NO: 4),
CDR2 of the heavy chain: GINPTSGGSNFNEKFKT (SEQ ID NO: 5),
CDR3 of the heavy chain: QGLWFDSDGRGFDF (SEQ ID NO: 6).

Further provided herein is a method of predicting whether a patient suffering from pancreatic ductal adenocarcinoma (PDAC) will be nonresponsive to treatment with a monoclonal antibody or antigen binding fragment thereof as defined herein. According to said method, the presence or absence of a KRAS, HRAS or NRAS mutation in a PDAC tumor in the patient is detected. Presence of a KRAS, HRAS or NRAS mutation indicates that the patient will be nonresponsive to treatment with said antibody.

The present invention further relates to a method of predicting whether a pancreatic ductal adenocarcinoma (PDAC) tumor will be nonresponsive to treatment with a monoclonal antibody or antigen binding fragment or peptide thereof as defined herein. This is accomplished by determining the presence or absence of a KRAS, HRAS or NRAS mutation in said PDAC tumor. Presence of a KRAS, HRAS or NRAS mutation indicates that the tumor will be nonresponsive to treatment with said antibody.

In a further aspect, the present invention relates to a method of treating a population of patients having been diagnosed with pancreatic ductal adenocarcinoma (PDAC), wherein the pancreatic ductal adenocarcinoma expresses the wild-type protein of at least one member of the RAS gene family selected from the KRAS wild-type, the HRAS wild-type or the NRAS wild-type, the method comprising administering to the patient a nucleic sequence encoding a monoclonal antibody or an antigen binding fragment or peptide thereof which specifically binds epidermal growth factor receptor (EGF-R), wherein the antibody is a chimeric or a humanized monoclonal antibody which contains the following complementarity determining regions (CDRs) :
CDR1 of the light chain: RSSQNIVHSNGNTYLD (SEQ ID NO: 1),
CDR2 of the light chain: KVSNRFS (SEQ ID NO: 2),
CDR3 of the light chain: FQYSHVPWT (SEQ ID NO: 3),
CDR1 of the heavy chain: NYYIY (SEQ ID NO: 4),
CDR2 of the heavy chain: GINPTSGGSNFNEKFKT (SEQ ID NO: 5),
CDR3 of the heavy chain: QGLWFDSDGRGFDF (SEQ ID NO: 6).

### DESCRIPTION OF THE FIGURES

The invention will be better understood with reference to the detailed description when considered in conjunction with the non-limiting examples and the accompanying drawings. The figures illustrate embodiments of methods of the invention.

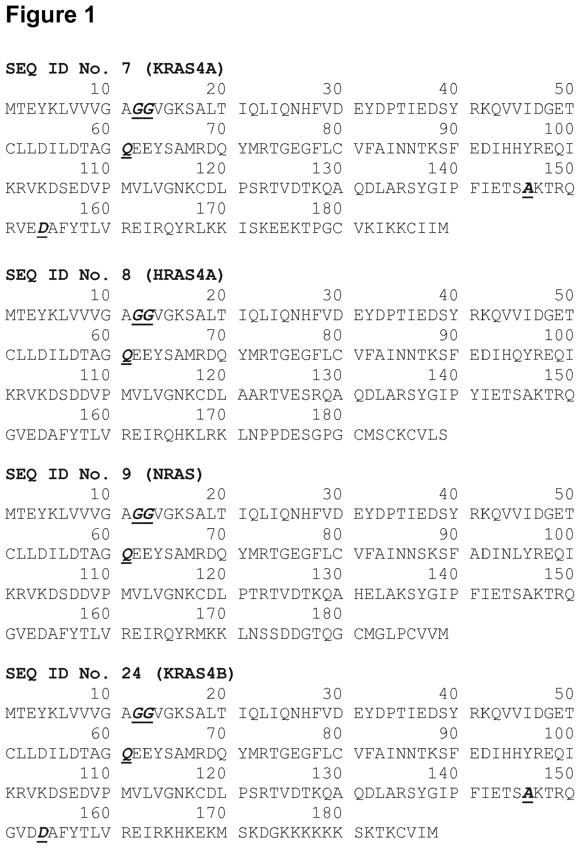

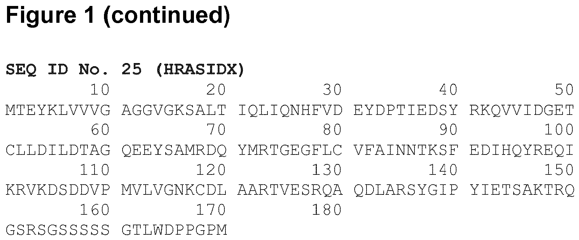

**Figure 1** shows the polypeptide sequence of wild-type KRAS, NRAS and HRAS: In bold, underlined italics, mutations affecting the responsiveness of a patient suffering from pancreatic ductal adenocarcinoma (PDAC) to treatment with a monoclonal antibody which specifically binds epidermal growth factor receptor (EGFR), or antigen binding fragment or peptide thereof as described herein.

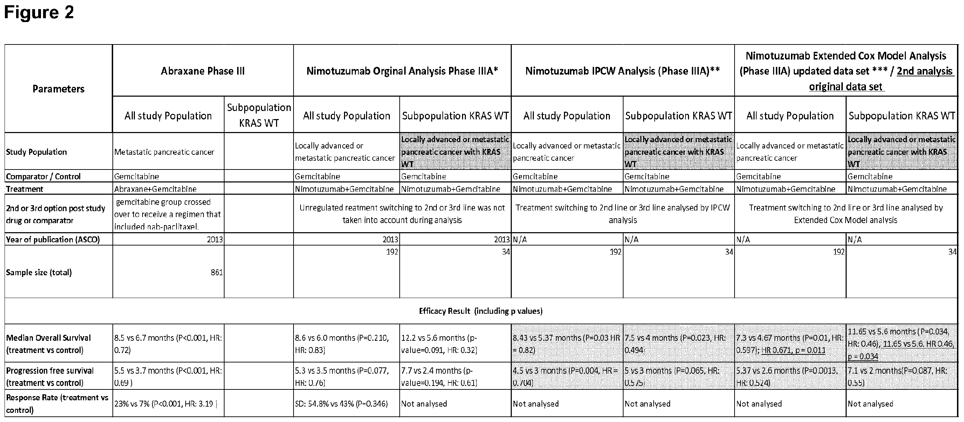

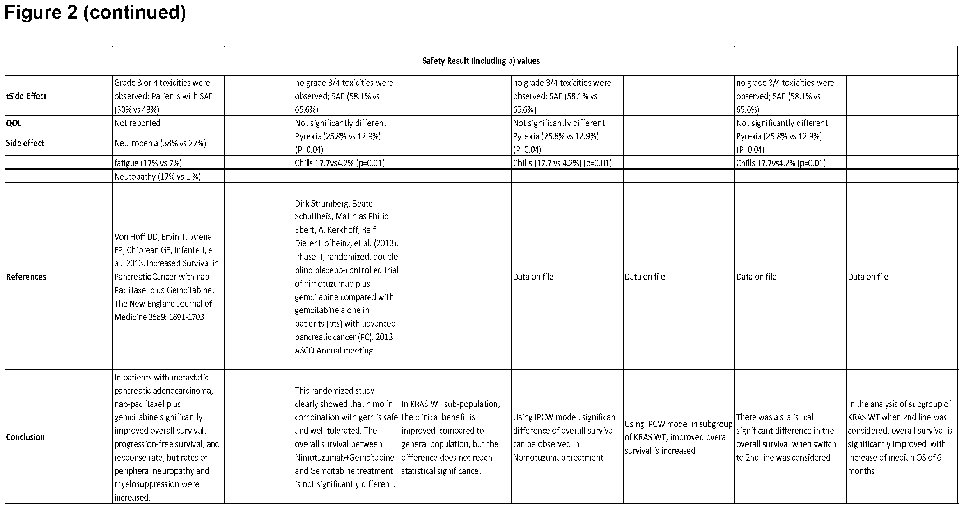

**Figure 2** shows the result of the evaluation of a clinical trial. In this clinical trial patients with previously untreated, unresectable, locally advanced or metastatic pancreatic adenocarcinoma received gemcitabine (1000 mg/m²/30-min iv once weekly (d1, 8, 15; q28)) and nimotuzumab (400 mg 30-min iv once weekly), or placebo until disease progression or unmanageable toxicity. The primary endpoint in this study was overall survival (OS) in the intention-to-treat (ITT) population. The secondary endpoints included progression free survival (PFS), safety, objective response rate (ORR), and Quality of Life (QoL).

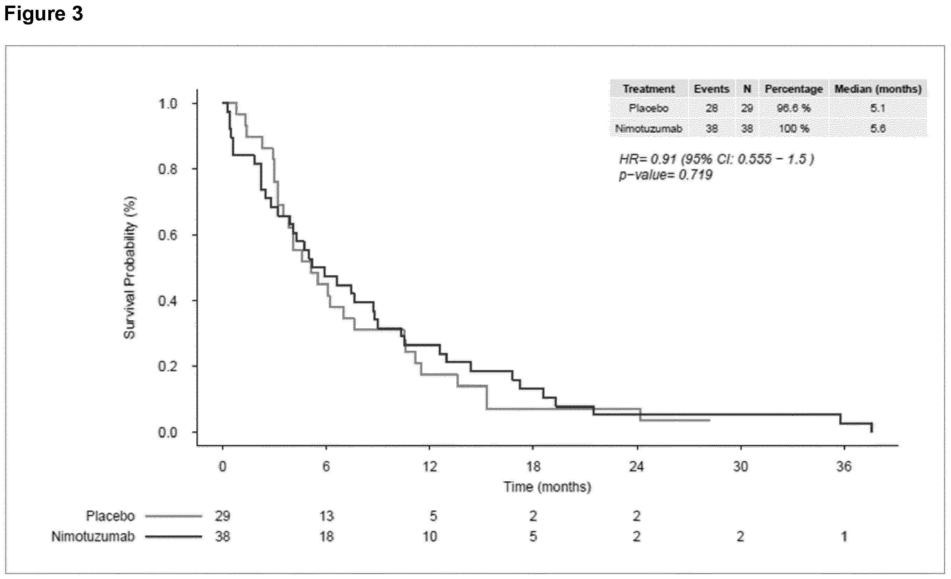

**Figure 3** shows Kaplan-Meier curves of the locally advanced or metastatic pancreatic cancer patients with mutated KRAS treated with placebo-gemcitabine compared to the patients treated with nimotuzumab-gemcitabine.

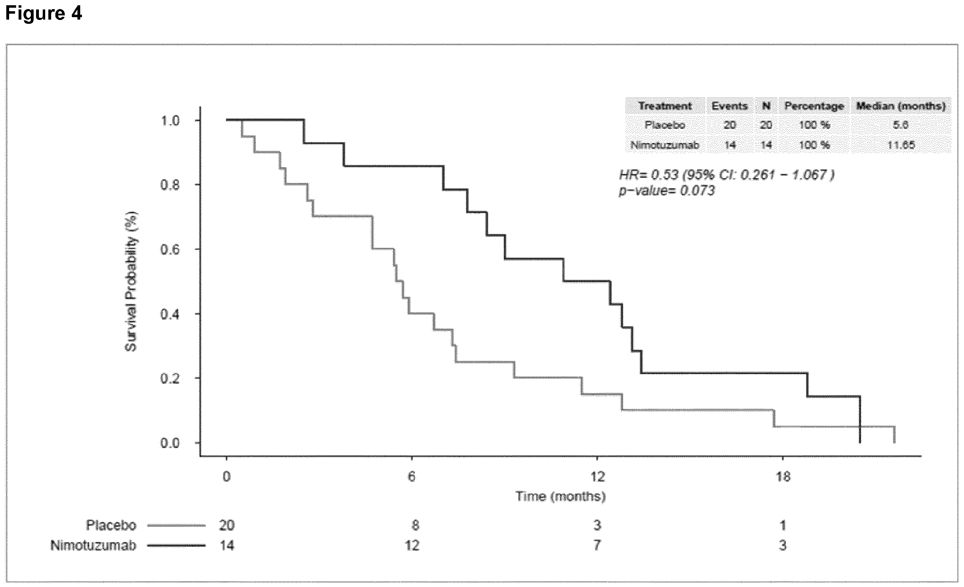

**Figure 4** shows Kaplan-Meier curves of the locally advanced or metastatic pancreatic cancer patient with wild-type KRAS treated with placebo-gemcitabine compared to the patients treated with nimotuzumab-gemcitabine. Treatment of patients with wild-type KRAS using nimotuzumab-gemcitabine resulted in a significant improvement in terms of progression-free-survival (PFS) and overall survival (OS).

### DETAILED DESCRIPTION

The present inventors provide herein, for the first time, a monoclonal antibody, or an antigen binding fragment or peptide thereof which specifically binds epidermal growth factor receptor (EGF-R) for use in a specific and effective treatment for a population of PDAC patients that has not been explicitly addressed in previous treatment approaches before. Herein, a novel use of a monoclonal antibody, or an antigen binding fragment or peptide thereof which specifically binds epidermal growth factor receptor (EGF-R) in a method of treating PDAC patients whose tumors express wild-type KRAS, wild-type HRAS or wild-type NRAS is provided. The inventors have surprisingly discovered that the anti-EGFR antibody nimotuzumab has a beneficial therapeutic effect on patients suffering from such PDAC tumors. This was unexpected, as no or only minimal therapeutic benefits could be found in patients whose PDAC tumors expressed mutated KRAS (HRAS, NRAS), which by far constitute the majority of all PDAC patients. Without wishing to be bound by theory, it is contemplated that those patients with KRAS, HRAS or NRAS mutated genes will not respond to the treatment with the anti-EGFR monoclonal antibody nimotuzumab, because the gene is activated, while patients with KRAS, HRAS or NRAS wilt type gene will respond to the therapy with anti-EGFR monoclonal antibody nimotuzumab.

In accordance with the foregoing, the present invention provides a monoclonal antibody, or an antigen binding fragment or peptide thereof which specifically binds epidermal growth factor receptor (EGF-R) for use in a method of treating a population of patients having been diagnosed with pancreatic ductal adenocarcinoma (PDAC), wherein the pancreatic ductal adenocarcinoma expresses the wild-type protein of at least one member of the RAS gene family selected from the KRAS wild-type, the HRAS wild-type or the NRAS wild-type, the method comprising administering to the patient a monoclonal antibody or an antigen binding fragment or peptide thereof which specifically binds epidermal growth factor receptor (EGF-R), wherein the antibody is a chimeric or a humanized monoclonal antibody which contains the following complementarity determining regions (CDRs) :
CDR1 of the light chain: RSSQNIVHSNGNTYLD (SEQ ID NO: 1),
CDR2 of the light chain: KVSNRFS (SEQ ID NO: 2),
CDR3 of the light chain: FQYSHVPWT (SEQ ID NO: 3),
CDR1 of the heavy chain: NYYIY (SEQ ID NO: 4),
CDR2 of the heavy chain: GINPTSGGSNFNEKFKT (SEQ ID NO: 5),
CDR3 of the heavy chain: QGLWFDSDGRGFDF (SEQ ID NO: 6).

Also described herein is the treatment said population of PDAC patients by administration of a nucleic acid encoding a monoclonal antibody or an antigen binding fragment or peptide thereof as described herein, i.e. which specifically binds epidermal growth factor receptor (EGF-R), wherein the antibody is a chimeric or a humanized monoclonal antibody which contains the following complementarity determining regions (CDRs) :
CDR1 of the light chain: RSSQNIVHSNGNTYLD (SEQ ID NO: 1),
CDR2 of the light chain: KVSNRFS (SEQ ID NO: 2),
CDR3 of the light chain: FQYSHVPWT (SEQ ID NO: 3),
CDR1 of the heavy chain: NYYIY (SEQ ID NO: 4),
CDR2 of the heavy chain: GINPTSGGSNFNEKFKT (SEQ ID NO: 5),
CDR3 of the heavy chain: QGLWFDSDGRGFDF (SEQ ID NO: 6).

What is disclosed in the context of treatment of patients with the monoclonal antibody (fragment/peptide) in the following also applies to treatment with the nucleic acid encoding said monoclonal antibody (fragment/peptide), *mutatis mutandis.*

### PDAC

The inventive uses relate to the treatment of pancreatic ductal adenocarcinoma, a pancreatic cancer. "Pancreatic cancer" is typically characterized by the presence of cancerous pancreatic cells and/or growth of a tumor mass in the pancreas. It is thus a physiological condition in a subject that is typically characterized by malignant cell growth in the pancreas. "Malignant" cells are not self-limited in their growth, are capable of invading into adjacent tissues, and may be capable of spreading to distant tissues (metastasizing). Malignant when used herein is synonymous with cancerous. "PDAC", or "pancreatic ductal adenocarcinoma", is a form of pancreatic cancer characterized by malignant cells derived from the pancreatic ductal epithelium.

### KRAS

The uses of the present invention are envisaged for treatment and/or stratification of PDAC patients whose PDAC tumors express a wild-type protein of at least one member of the RAS gene family. Said protein may be "wild-type" KRAS. The term "wild-type" KRAS refers to the naturally occurring KRAS isoforms (Uniprot Acc. No. P01116, version 190 of July 22, 2015). It is in particular envisaged that wild-type KRAS comprises a sequence corresponding to SEQ ID NO.: 7 or SEQ ID No. 24 . The wild-type KRAS may consist of a sequence corresponding to SEQ ID NO.: 7 or SEQ ID No. 24.

The term "wild-type KRAS" generally also encompasses KRAS polypeptides having mutations relative to the reference sequence shown in SEQ ID NO: 7 (KRAS4A) or SEQ ID No. 24 (KRAS4B) and also encompasses polypeptides having an amino acid sequence which shares as a certain degree of identity with the amino acid sequence shown in SEQ ID NO: 7 (KRAS4A, Uniprot Acc. No. P01116-1) or SEQ ID No. 24 (KRAS4B, Uniprot Acc. No. P01116-2) as described herein. More specifically, "wild-type KRAS" includes KRAS polypeptides having at least 80%, 85%, 90%, 95% or 100% identity with SEQ ID NO.: 7 (KRAS4A) or SEQ ID No. 24 (KRAS4B).In particular, KRAS isoforms 4A and 4B are encompassed by the term "wild-type KRAS". Preferably, the "wild-type KRAS" does not comprise a mutation in its amino acid sequence compared to SEQ ID No. 7 or SEQ ID No. 24, respectively. Preferably, the wild-type KRAS does not comprise a mutation in any of amino acid residues of codon G12, G13, Q61, A146 or D154 of SEQ ID NO.: 7 or SEQ ID No. 24. The lacking mutation, i.e. the mutation not present in the wild-type KRAS amino acid sequence, may in particular be G12D, G12A, G12R, G12C, G12S, G12V and G13D.

The wild-type KRAS is preferably characterized in that it is capable of turning off the signaling pathways by catalyzing hydrolysis of guanosine triphosphates (GTP) to guanosine diphosphates. GTPase activity (i.e., capability of GTP hydrolysis to guanosine diphosphates) can be readily assessed using commercially available GTPase assay kits.

### HRAS

The PDAC tumors of the patients to be treated and/or stratified according to the uses of the invention may express "wild-type" HRAS. The term "wild-type" HRAS refers to the naturally occurring HRAS (Uniprot Acc. No. P01112, version 202 of July 22, 2015). It is in particular envisaged that wild-type HRAS comprises a sequence corresponding to SEQ ID NO.: 8 or SEQ ID No. 25. The wild-type HRAS may consist of a sequence corresponding to SEQ ID NO.:8 or SEQ ID No. 25.

The term "wild-type HRAS" generally also encompasses HRAS polypeptides having mutations relative to the reference sequence shown in SEQ ID NO: 8 or SEQ ID No. 25 and also encompasses polypeptides having an amino acid sequence which shares as a certain degree of identity with the amino acid sequence shown in SEQ ID NO: 8 or SEQ ID No. 25 as described herein. More specifically, "wild-type HRAS" includes HRAS polypeptides having at least 80%, 85%, 90%, 95% or 100% identity with SEQ ID NO.: 8 or SEQ ID No. 25. In particular, isoforms H-Ras4A (Uniprot Acc. No. P01112-1) and H-Ras (Uniprot Acc. No. P01112-2) are encompassed by the term "wild-type HRAS". Preferably, the "wild-type HRAS" does not comprise a mutation in any of amino acid residues of does not comprise a mutation in any of amino acid residues of codon G12, G13, or Q61 of SEQ ID NO.: 8 or SEQ ID No. 25.

### NRAS

The member of the RAS gene family which is expressed "wild-type" in the PDAC tumors of the patients to be treated and/or stratified according to the methods and uses of the invention may, additionally or alternatively, be NRAS. The term "wild-type" NRAS refers to the naturally occurring NRAS (Uniprot Acc. No. P01111, version 182 of July 22, 2015). It is in particular envisaged that wild-type NRAS comprises a sequence corresponding to SEQ ID NO.: 9. The wild-type NRAS may consist of a sequence corresponding to SEQ ID NO.: 9.

The term "wild-type NRAS" generally also encompasses NRAS polypeptides having mutations relative to the reference sequence shown in SEQ ID NO: 9 and also encompasses polypeptides having an amino acid sequence which shares as a certain degree of identity with the amino acid sequence shown in SEQ ID NO: 9 as described herein. More specifically, "wild-type NRAS" includes NRAS polypeptides having at least 80%, 85%, 90%, 95% or 100% identity with SEQ ID NO.: 9. Preferably, the "wild-type NRAS" does not comprise a mutation in any of amino acid residues of does not comprise a mutation in any of amino acid residues of codon G12, G13, or Q61 of SEQ ID NO.: 9.

"Sequence identity" or "% identity" refers to the percentage of residue matches between at least two polypeptide or polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Sequence comparisons can be performed using standard software programs such as the NCBI BLAST program.

In the context of the invention, the expression "position corresponding to another position" (e.g., regions, fragments, nucleotide or amino acid positions, or the like) is based on the convention of numbering according to nucleotide or amino acid position number and then aligning the sequences in a manner that maximizes the percentage of sequence identity. Because not all positions within a given "corresponding region" need be identical, non-matching positions within a corresponding region may be regarded as "corresponding positions." Accordingly, as used herein, referral to an "amino acid position corresponding to amino acid position [X]" of a specified protein sequence represents, in addition to referral to amino acid positions of the specified protein sequence, referral to a collection of equivalent positions in other recognized protein and structural homologues and families.

### Anti-EGFR antibody

The present inventors have discovered the therapeutic benefit of using a monoclonal antibody (or an antigen-binding fragment or peptide thereof) as defined herein for treating patients with a PDAC tumor expressing wild-type KRAS, HRAS or NRAS.

As is well known in the art, an antibody is an immunoglobulin molecule capable of specific binding to a target through at least one epitope recognition site, located in the variable region of the immunoglobulin molecule. Antigen-binding fragments are also envisaged for use in the methods of the invention. The term "antigen-binding fragment" as used herein refers to a polypeptide fragment that contains all 6 CDRs of a VH and VL sequence set forth herein from monoclonal antibodies that specifically bind EGFR. The term comprises dAb, Fab, Fab', F(ab')₂, Fv), single chain Fvs (ScFv), diabodies, and minibodies comprising a scFv joined to a CH3 domain. Also included are synthetic variants, naturally occurring variants, fusion proteins comprising an antibody portion with an antigen-binding fragment of the required specificity and any other modified configuration of the immunoglobulin molecule that comprises an antigen-binding site or fragment (epitope recognition site) of the required specificity.

The monoclonal antibody (or antigen-binding fragment thereof) used in the methods of the invention is also termed "anti-EGFR antibody" herein. The term "anti-EGFR antibody" is used herein to denote the antibodies potential to specifically bind to EGFR. The expression "antibody (fragment/peptide)" is used herein as an abbreviation to denote the monoclonal antibody or antigen-binding fragment or peptide thereof as applied in the methods of the invention. "EGFR" or "EGF-R" refers to the epidermal growth factor receptor.

The monoclonal antibody or antigen-binding fragment or peptide thereof for use in accordance with the methods and uses of the invention is envisaged to specifically bind to epidermal growth factor receptor (EGF-R). Binding to EGF-R can be assessed by routine methods, e.g. by adding the antibody (fragment/peptide) to plates coated with (partially) purified EGFR and detecting antibody (fragment/peptide) binding to the EGFR by a solid phase, indirect immune-enzymatic assay as briefly described in European Patent EP 0586 002 B2. "Specifically binding" refers to the ability of a specific binding agent (here: the anti-EGFR antibody or an antigen-binding fragment or peptide thereof) to bind to its target (here: EGFR) with a greater affinity than it binds to a non-target. Binding affinity may for example be determined by an affinity ELISA, a BIAcore assay, a kinetic method, or an equilibrium/solution method. The anti-EGFR antibody (fragment/peptide) may be defined as "specifically binding" to EGFR when the dissociation constant between the antibody and its recognized epitope is less than 1 µM, preferably less than 100 nM and more preferably less than 10 nM. Preferably, EGFR-binding characteristics of the antibody (fragment/peptide) used in the methods of the invention are comparable or equal to the EGFR-binding characteristics of the antibody disclosed in European Patent EP 0 586 002 B1 as ascertainable by the indirect immune-enzymatic assay described therein.

It is envisaged that the anti-EGFR antibody (fragment/peptide) may recognize or bind to both human EGFR present in normal, i.e. non-tumoral cells ("normal EGFR") and EGFR present in tumoral cells ("tumoral EGFR"). Binding to both normal and tumoral EGFR can be easily assessed by using soluble EGFR obtained from non-malignant cells and EGFR obtained from malignant cells in the indirect immune-enzymatic assay described in European Patent EP 0 586 002 B1, respectively.

### Chimeric antibody

The monoclonal antibody applied in the methods of the invention may be a chimeric antibody (or antigen-binding fragment or peptide thereof). The term "chimeric antibody" refers to an antibody in which a portion of the heavy and/or light chain is identical with or homologous to corresponding sequences in antibodies derived from a particular species or belonging to a particular antibody class or subclass, while the remainder of the chain (s) is identical with or homologous to corresponding sequences in antibodies derived from another species or belonging to another antibody class or subclass, as well as fragments of such antibodies, as long as they exhibit the desired biological activity, i.e. (specifically) bind to EGFR.

It is envisaged that the chimeric antibody may comprise
a variable region of the light chain corresponding to DVLMTQIPLSLPVSLGDQASISCRSSQNIVHSNGNTYLDWYLQKPGQSPNLLIYKVS NRFSGVPDRFRGSGSGTDFTLKISRVEAEDLGVYYCFQYSHVPWTFGGGTKLEIKR A (SEQ ID NO.: 10) and a variable region of the heavy chain corresponding to QVQLQQPGAELVKPGASVKLSCKASGYTFTNYYIYWVKQRPGQGLEWIGGINPTSG GSNFNEKFKTKATLTVDESSTTAYMQLSSLTSEDSAVYYCTRQGLWFDSDGRGFDF WGQGTTLTVSS (SEQ ID NO.: 11).

### Humanized antibody

"Humanized antibodies" contain minimal sequence derived from the non-human antibody. In general, humanized antibodies are human immunoglobulins in which residues from a hypervariable region of the recipient are replaced by residues from a hypervariable region of a non-human species such as mouse, rat, rabbit or non-human primate having the desired biological effect, i.e. specifically bind to EGFR in the context of the present invention. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin.

Particularly envisaged humanized monoclonal antibodies (or antigen-binding fragments) for use in the methods of the invention comprise the following variable regions:
a variable region of the light chain corresponding to DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITRE (SEQ ID NO.: 12) and a variable region of the heavy chain corresponding to QVQLQQSGAEVKKPGSSVKVSCKASGYTFTNYYIYWVRQAPGQGLEWIGGINPTS GGSNFNEKFKTX¹X²TITVDESX³X⁴TAYMELSSLRSEDTAFYFCX⁵RQGLWFDSDGR GFDFWGQGSTVTVSS, wherein X¹ is R or K; X² is V or A; X³ is T or S; X⁴ is N or T; and X⁵ is A or T (SEQ ID NO.: 13); or
a variable region of the light chain corresponding to DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITRE (SEQ ID NO.: 14) and a variable region of the heavy chain corresponding to QVQLQQSGAEVKKPGSSVKVSCKASGYTFTNYYIYWVRQAPGQGLEWIGGINPTS GGSNFNEKFKTRVTITVDESSTTAYMELSSLRSEDTAFYFCTRQGLWFDSDGRGFD FWGQGSTVTVSS (SEQ ID NO.: 15); or
a variable region of the light chain corresponding to DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITRE (SEQ ID NO.: 16) and a variable region of the heavy chain corresponding to QVQLQQSGAEVKKPGSSVKVSCKASGYTFTNYYIYWVRQAPGQGLEWIGGINPTS GGSNFNEKFKTKATITVDESSTTAYMELSSLRSEDTAFYFCTRQGLWFDSDGRGFD FWGQGSTVTVSS (SEQ ID NO.: 17); or
a variable region of the light chain corresponding to DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITRE (SEQ ID NO.: 18) and a variable region of the heavy chain corresponding to QVQLQQSGAEVKKPGSSVKVSCKASGYTFTNYYIYWVRQAPGQGLEWIGGINPTS GGSNFNEKFKTRVTITVDESSTTAYMELSSLRSEDTAFYFCARQGLWFDSDGRGFD FWGQGSTVTVSS (SEQ ID NO.: 19); or
a variable region of the light chain corresponding to DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITRE (SEQ ID NO.: 20), and a variable region of the heavy chain corresponding to QVQLQQSGAEVKKPGSSVKVSCKASGYTFTNYYIYWVRQAPGQGLEWIGGINPTS GGSNFNEKFKTRVTITVDESTNTAYMELSSLRSEDTAFYFCTRQGLWFDSDGRGFD FWGQGSTVTVSS (SEQ ID NO.: 21); or
a variable region of the light chain corresponding to DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITRE (SEQ ID NO.: 22) and a variable region of the heavy chain corresponding to QVQLQQSGAEVKKPGSSVKVSCKASGYTFTNYYIYWVRQAPGQGLEWIGGINPTS GGSNFNEKFKTKATITVDESTNTAYMELSSLRSEDTAFYFCTRQGLWFDSDGRGFD FWGQGSTVTVSS (SEQ ID NO.: 23).

It is further envisaged for all the monoclonal antibodies (and, where applicable, antigen-binding fragments or peptides thereof) described herein to comprise a constant regions of the heavy chain comprising the amino acid sequence of a gamma-1 chain of a human immunoglobulin and the constant regions of the light chain of said antibody comprise the amino acid sequence of a kappa chain of a human immunoglobulin.

### Nimotuzumab

The present inventors have found that the monoclonal antibody (or antigen-binding fragment thereof) produced by the cell line h-R3 deposited as ECACC 951110101 is particularly useful for application in the invention. In particular, the monoclonal antibody may be nimotuzumab ((Ramakrishnan et al. MAbs. 2009 Jan-Feb; 1(1): 41-48.).

### Modifications

The antibodies and antigen-binding fragments thereof used in the methods of the invention may be modified. Typical modifications conceivable in the context of the invention include, e.g., chemical modifications as described in the following.

Generally, all kind of modifications are conceivable as long as they do not alter the CDR sequences of said antibody or antigen-binding fragment as defined elsewhere herein and further do not abolish the capability of monoclonal antibody (or antigen-binding fragment or peptide thereof) to specifically bind to EGFR.

Possible chemical modifications of the monoclonal antibody (or antigen-binding fragment thereof) include acylation or acetylation of the amino-terminal end or amidation or esterification of the carboxy-terminal end or, alternatively, on both. The modifications may also affect the amino group in the side chain of lysine or the hydroxyl group of threonine. Other suitable modifications include, e.g., extension of an amino group with polypeptide chains of varying length (e.g., XTEN technology or PASylation®), N-glycosylation, O-glycosylation, and chemical conjugation of carbohydrates, such as hydroxyethyl starch (e.g., HESylation®) or polysialic acid (e.g., PolyXen® technology). Chemical modifications such as alkylation (e. g., methylation, propylation, butylation), arylation, and etherification may be possible and are also envisaged.

### Functional features

It is also envisaged that the monoclonal antibody (or antigen-binding fragment or peptide thereof) may have one or more of the following capabilities:

The anti-EGFR antibody (fragment/peptide) may be capable of inhibiting binding of EGF to the receptor, as readily ascertainable by the radioligand competition assay as described in Mateo et al. Immunotechnology. 1997 Mar;3(1):71-81.

The anti-EGFR antibody (fragment/peptide) may be capable of inhibiting growth of EGF-dependent tumor cells, as readily ascertainable by, e.g. cultivating EGF-dependent tumor cell lines (e.g., U-1752 and H125) in the presence of the antibody (fragment/peptide) and, after a predetermined period of time, quantify the total number of cells, e.g. by determination of total protein. An exemplary method is described in EP0586002.

The anti-EGFR antibody (fragment/peptide) may elicit antibody-dependent cellular cytotoxicity (ADCC). Several routine methods exist for determining the efficacy of antibodies (fragments) in eliciting ADCC, including the chromium-51 release assay, europium release assay, and sulfur-35 release assay. Briefly, a labelled target cell line expressing EGFR is incubated with the anti-EGFR antibody (fragment/peptide). After washing, effector cells (for example, peripheral blood mononuclear cells (PBMC) obtained from donors) expressing Fc receptor CD16 are co-incubated with the antibody-labelled target cells. Target cell lysis is subsequently measured by release of intracellular label by a scintillation counter or spectrophotometry. An exemplary protocol is disclosed in EP0586002 B1.

The anti-EGFR antibody (fragment/peptide) may have an anti-angiogenic effect. Means and methods for assessing angiogenesis in vitro and in vivo have been reviewed by Staton et al. Int J Exp Pathol. 2004 Oct; 85(5): 233-248. E.g., assays of endothelial cell proliferation are readily available and easy to perform. Briefly, endothelial cells are e.g. incubated with a candidate anti-EGFR antibody (fragment/peptide). After incubation, the net cell number is determined using e.g. an electronic counter such as a Coulter counter and compared to untreated controls.

The anti-EGFR antibody (fragment/peptide) may have an anti-apoptotic effect. A variety of apoptosis kits are commercially available from various manufacturers and evaluate, e.g., active Caspase-3, -7 or -9, DNA fragmentation, phosphatidyl serine (PS) on the cytoplasmic surface of the cell membrane, membrane integrity and other apoptosis markers.

The anti-EGFR antibody (fragment/peptide) may cause an effect on CD133 positive cancer stem cells. CD133-positive cells display many cancer-stem cell characteristics, such as tumorsphere formation ability, expression of early-stage development markers, high invasiveness, raised tumor initiation potential and resistance to cisplatin chemotherapy treatment. It is envisaged that the anti-EGFR antibody (peptide/fragment) of the invention may reduce or abolish any of the cancer-stem cell properties.

The anti-EGFR antibody (fragment/peptide) may elicit complement-mediated cytotoxic activity, which can be assessed similarly to ADCC as described above. However, instead of effector cells, a complement source (e.g. rabbit serum) is added to the target cells, and target cell lysis is measured by release of intracellular label as described in EP0586002.

The anti-EGFR antibody (fragment/peptide) may induce cell-cyle arrest as easily ascertainable using flow cytometry. Briefly, cells are permeabilised and treated with a fluorescent dye that stains DNA quantitatively, usually propidium iodide (PI). As the DNA content of cells duplicates during the S phase of the cell cycle, the relative amount of cells in the G0 phase and G1 phase (before S phase), in the S phase, and in the G2 phase and M phase (after S phase) can be determined, as the fluorescence of cells in the G2/M phase will be twice as high as that of cells in the G0/G1 phase.

The anti-EGFR antibody (fragment/peptide) may be capable of producing a synergetic effect in inhibiting proliferation of tumor cells if combined with N-acetyl GM3 ganglioside, as readily ascertainable by culturing EGFR-expressing cell lines (such as H-125) in the presence of the anti-EGFR antibody (fragment/peptide) and N-acetyl GM3, and determine total cell numbers (e.g. by assessing total protein concentration) after a predefined period of time in relation to untreated controls. It is envisaged that the synergistic effect of the anti-EGFR antibody (fragment/peptide) and N-acetyl GM3 results in an inhibition of EGFR-expressing tumor cell proliferation. Again, a protocol for assessing said synergistic effect is provided in EP0586002.

The anti-EGFR antibody (fragment/peptide) may be capable of producing a synergetic effect in inhibiting proliferation of tumor cells if combined with a monoclonal antibody against EGF, as can be easily evaluated using a similar protocol as for determination of the synergistic effect of the anti-EGFR antibody (fragment/peptide) and N-acetyl GM3; with the exception that the cells are cultivated in the presence of an anti-EGF antibody instead of N-acetyl GM3. Again, it is envisaged that the synergistic effect of the anti-EGFR antibody (fragment/peptide) and anti-EGF antibody results in an inhibition of EGFR-expressing tumor cell proliferation.

The anti-EGFR antibody (fragment/peptide) may be capable of repairing tumor microsatellite instability produced by radiotherapy. Microsatellite instability (MSI) is a hypermutable phenotype caused by the loss of DNA mismatch repair activity and can be assessed by evaluating microsatellite markers as suggested by Dietmaier et al. Cancer Res. 1997 Nov 1;57(21):4749-56.

### Patients

Whereas most of the PDAC studies relate to patients with mutated KRAS, the present inventors have, for the first time, developed an effective treatment for a population of patients with PDAC expressing wild-type KRAS, HRAS or NRAS. The term "patient" refers to a human patient preferably being pre-diagnosed with PDAC, in particular locally advanced and/or metastatic PDAC.

Whether or not a certain patient belongs to the population of patients to be treated by the inventive method can be assessed using routine experimentation known in the art. E.g., in order to determine whether or not a PDAC tumor expresses wild-type KRAS, HRAS or NRAS a tumor sample is typically obtained from the patient to be evaluated, and KRAS, HRAS or NRAS nucleic acid sequence is, respectively, obtained from the sample and amplified, and subjected to sequencing.

Another method is related to plasma or serum samples wherein circulating tumor cells with KRAS, HRAS or NRAS oncogene can be detected using membrane microarrays. In this regards a positive *KRAS, HRAS or NRAS* mutation in plasma or serum suggests a *KRAS, HRAS or NRAS* mutation in the tumor whereas the absence of a *KRAS, HRAS or NRAS* mutation in the plasma or serum does not necessarily prove a lack of a similar mutation in the pancreatic tumor tissue.

Additionally or alternatively, expression of wild-type KRAS, HRAS or NRAS can be determined by detecting mutant and/or wild-type KRAS, HRAS or NRAS polypeptides in the tumor sample, e.g. by using specific antibodies binding to epitopes specific for wild-type or mutant KRAS, HRAS or NRAS, respectively. It is envisaged that patients with PDAC tumors expressing wild-type KRAS, HRAS or NRAS are selected for treatment, whereas patients expression mutant KRAS, HRAS or NRAS are not selected for treatment.

It is thought that patients with PDAC tumors expressing mutant KRAS, HRAS or NRAS and in particular KRAS, HRAS or NRAS having on or more mutations as set out elsewhere herein, are less responsive or non-responsive to treatment with the monoclonal antibody (fragment/peptide) used in the methods of the invention. Thus, the present invention also provides a method for stratification of PDAC patients. "Stratification", when used herein, means sorting PDAC patients into those who may benefit from treatment with the anti-EGFR antibody (or antigen-binding fragment or peptide thereof) as defined herein, and those who may not benefit. It is envisaged that patients whose PDAC tumors do not express wild-type KRAS, HRAS or NRAS and in particular patients whose PDAC tumors express mutated KRAS, HRAS or NRAS, are not likely to benefit from (be responsive to) treatment with the anti-EGFR antibody (or antigen-binding fragment or peptide thereof). Said mutations which presence is in especially thought to be indicative for non-responsiveness to treatment with an anti-EGFR antibody (fragment/peptide) according to the invention are, are G12, G13, Q61, A146 or D154 of SEQ ID NO.: 7 or SEQ ID No. 24 (KRAS); G12, G13, or Q61 of SEQ ID NO.: 8 or SEQ ID No. 25 (HRAS) or G12, G13, or Q61 of SEQ ID NO.: 9 (NRAS).

Otherwise, patients whose PDAC tumors express wild-type KRAS, HRAS or NRAS as defined elsewhere herein are likely to benefit (be responsive to) from the treatment as described herein. Said "wild-type" KRAS preferably has a sequence corresponding to SEQ ID No. 7 or SEQ ID No. 24 and preferably does not comprise mutations in amino acid residues G12, G13, Q61, A146 or D154 of SEQ ID NO.: 7 or SEQ ID No. 24, said "wild-type" HRAS preferably has a sequence corresponding to SEQ ID No. 8 or SEQ ID No. 25 and preferably does not comprise mutations in amino acid residues G12, G13, or Q61 of SEQ ID No. 8 or SEQ ID No. 25, and/or said "wild-type" NRAS preferably has a sequence corresponding to SEQ ID No. 9 and preferably does not comprise mutations in amino acid residues G12, G13, or Q61 of SEQ ID No. 9.

The term "being responsive" in the context of the method of treatment provided herein means that a patient, or tumor, or tumor shows a complete response or a partial response after administering the anti-EGFR antibody (fragment/peptide) as defined herein, according to RECIST (Response Evaluation Criteria in Solid Tumors). The term "nonresponsive" as used herein means that a patient or tumor shows stable disease or progressive disease after administering the anti-EGFR antibody (fragment/peptide) as defined herein, according to RECIST. RECIST is described, e.g., in Eisenhauer et al.: New response evaluation criteria in solid tumours: revised RECIST guideline (version 1.1). Eur. J. Cancer. 45, Nr. 2, 2009, 228-47.

Also provided herein is therefore a method of selecting a patient or the population of patients to be treated. This is achieved by determining the presence or absence of mutant and/or wild-type KRAS, HRAS or NRAS in a PDAC tumor sample of each patient. Patients, in whose tumor samples mutant KRAS, HRAS or NRAS is detected and/or wild-type KRAS, HRAS or NRAS is not detected are not considered eligible for treatment, whereas patients in whose tumor samples wild-type KRAS, HRAS or NRAS is detected and/or mutant KRAS, HRAS or NRAS is not detected are considered eligible are therefore selected for treatment in accordance with the invention.

Further, as described before, the invention allows provides a method for prognosis of whether a patient suffering from PDAC, or a PDAC tumor, will be responsive or nonresponsive to treatment with the anti-EGFR antibody (fragment/peptide) as described herein. As set out before, absence or presence of mutated and/or wild-type KRAS, HRAS or NRAS in a tumor sample of the patient can be assessed using routine methods known in the art and described elsewhere herein. Expression of mutated KRAS, HRAS or NRAS in the PDAC tumor indicates that the patient, or PDAC tumor, will be nonresponsive to treatment, whereas expression of wild-type KRAS, HRAS or NRAS in the PDAC tumor indicates that the patient will be responsive to treatment with the antibody (fragment/peptide) as described herein.

### Treatment

The term "treatment" in all its grammatical forms includes therapeutic or prophylactic treatment of PDAC. A "therapeutic or prophylactic treatment" comprises prophylactic treatments aimed at the complete prevention of clinical and/or pathological manifestations or therapeutic treatment aimed at amelioration or remission of clinical and/or pathological manifestations. The term "treatment" thus also includes the amelioration or prevention of PDAC.

In the context with the present invention the term "therapeutic effect" in general refers to the desirable or beneficial impact of a treatment, e.g. amelioration or remission of the disease manifestations. The term "manifestation" of a disease is used herein to describe its perceptible expression, and includes both clinical manifestations, hereinafter defined as indications of the disease that may be detected during a physical examination and/or that are perceptible by the patient (i.e., symptoms), and pathological manifestations, meaning expressions of the disease on the cellular and molecular level. The therapeutic effect of treatment with the anti-EGFR antibody (fragment/peptide) can be assessed by RECIST as described elsewhere herein. Additionally or alternatively it is also possible to evaluate the general appearance of the respective patient (e.g., fitness, well-being) which will also aid the skilled practitioner to evaluate whether a therapeutic effect has been elicited. The skilled person is aware of numerous other ways which are suitable to observe a therapeutic effect of the compounds of the present invention.

### Dose

Preferably, a therapeutically effective amount of anti-EGFR antibody (fragment/peptide) is administered. By "therapeutically effective amount" is meant an amount of the monoclonal antibody (or antigen-binding fragment or fragment thereof) that elicits a therapeutic effect as described herein. The exact dose of anti-EGFR antibody (fragment/peptide) will depend on the purpose of the treatment (e.g. remission maintenance vs. acute tumor growth or metastatic spread), and will be ascertainable by one skilled in the art using known techniques. Adjustments for route of administration, age, body weight, general health, sex, diet, time of administration, drug interaction and the severity of the condition may be necessary, and will be ascertainable with routine experimentation by those skilled in the art.

Also provided herein is a treatment regimen for treating patients with PDAC with a monoclonal antibody (fragment/peptide), said antibody preferably being nimotuzumab. It is envisaged to treat patients with nimotuzumab in a dosage range of between 200 and 400 mg once a week. The envisioned dosage range of "between 200 mg and 400 mg" comprises, e.g., 200 mg, 250 mg, 300 mg, 350 mg and 400 mg and dosages in between. Weakly treatment with the dosage of nimotuzumab between 200 and 400 mg is envisioned to be carried out over a period of time of 12, 24 or 36 weeks. After said weakly treatment, patients may be treated with a dosage of nimotuzumab of between 200 and 400 mg once every 2-3 weeks.

### Administration

A variety of routes are applicable for administration of the monoclonal antibody (peptide/fragment) according to the present invention, including, but not limited to, orally, topically, transdermally, subcutaneously, intravenously, intraperitoneally, intramuscularly or intraocularly. However, any other route may readily be chosen by the person skilled in the art if desired. Intravenous infusion of said antibody (peptide/fragment) is particularly envisaged.

### Anti-cancer agents

Also envisaged herein is the additional administration (co-administration) of anti-cancer agents to PDAC patients. Said anti-cancer agents may be administered antecedently, simultaneously, and/or subsequently to the antibody (fragment/peptide) as described herein.

In the context of the present invention, the term "anti-cancer agent" comprises any agent useful in the treatment of PDAC, including chemotherapeutic agents, anti-neoplastic agents and biological molecules. Agents included in the definition of anti-cancer agents are, without limitation, alkylating agents, e.g. mechlorethamine, cyclophosphamide, melphalan, chlorambucil, ifosfamide, busulfan, N-Nitroso-N-methylurea (MNU), carmustine (BCNU), lomustine (CCNU), semustine (MeCCNU), fotemustine, streptozotocin, dacarbazine, mitozolomide, temozolomide, thiotepa, mytomycin, diaziquone (AZQ), cisplatin, carboplatin, oxaliplatin, procarbazine and hexamethylmelamine; antimetabolites, e.g. methotrexate, pemetrexed, fluorouracil, capecitabine, cytarabine, gemcitabine, decitabine, Vidaza, fludarabine, nelarabine, cladribine, clofarabine, pentostatin, thioguanine, mercaptopurine; anti-microtubule agents e.g. vincristine, vinblastine, vinorelbine, vindesine, vinflunine, paclitaxel, docetaxel, podophyllotoxin; topoisomerase inhibitors, e.g. irinotecan, topotecan, etoposide, doxorubicin, mitoxantrone, teniposide, novobiocin, merbarone, aclarubicin; cytotoxic antibiotics, e.g. actinomycin, bleomycin, plicamycin, mitomycin, doxorubicin, daunorubicin, epirubicin, idarubicin, pirarubicin, aclarubicin, and mitoxantrone. Particularly envisaged is the use of gemtabicine, FOLFIRINOX, erlotinib, 5-fluorouracil, paclitaxel, nab-paclitaxel, docetaxel, capecitabine, oxaliplatin cisplatin, FOLFOXIRI, abraxane, anti-CD40 antibodies, oregovomab, Nelfinavir, cetuximab, tegafur, leucovorin and combinations thereof.

Additional administration of palliative agents in order to reduce or control side effects of PDAC treatment and PDAC symptoms, including pain, nausea, breathlessness, insomnia, and other physical symptoms caused by cancer or its treatment is also envisaged.

### Composition

It is envisaged to administer the anti-EGFR antibody (fragment/peptide) or a nucleic acid encoding said anti-EGFR antibody or antigen-binding fragment or peptide thereof in the form of a pharmaceutical composition. Preferably, said anti-EGFR antibody (fragment/peptide) or nucleic acid is present in the pharmaceutical composition in a therapeutically effective amount. The term "pharmaceutical composition" particularly refers to a composition suitable for administering to a human, i.e., a composition containing components which are pharmaceutically acceptable. Preferably, a pharmaceutical composition comprises a monoclonal antibody (or antigen-binding fragment or peptide thereof) or nucleic acid as described herein together with one or more pharmaceutical excipients. The composition may also comprise further anti-cancer agents as described elsewhere herein. The term "excipient" includes fillers, binders, disintegrants, coatings, sorbents, antiadherents, glidants, preservatives, antioxidants, flavoring, coloring, sweeting agents, solvents, co-solvents, buffering agents, chelating agents, viscosity imparting agents, surface active agents, diluents, humectants. carriers, diluents, preservatives, emulsifiers, stabilizers or tonicity modifiers. Pharmaceutical compositions of the invention preferably comprise a therapeutically effective amount of the monoclonal antibody (or antigen-binding fragment or peptide thereof) and can be formulated in various forms, e.g. in solid, liquid, gaseous or lyophilized form and may be, inter alia, in the form of an ointment, a cream, transdermal patches, a gel, powder, a tablet, solution, an aerosol, granules, pills, suspensions, emulsions, capsules, syrups, liquids, elixirs, extracts, tincture or fluid extracts or in a form which is particularly suitable for the desired method of administration.

The pharmaceutical composition of the present invention may further comprise one or more additional anti-cancer agents. Preferably, said agents are therapeutically effective for treatment of PDAC. Particularly suitable anti-cancer agents have been described in the context of co-administration herein. Examples that can be used in the pharmaceutical composition of the invention include, without limitation, gemtabicine, FOLFIRINOX, erlotinib, 5-fluorouracil, paclitaxel, nab-paclitaxel, docetaxel, capecitabine, oxaliplatin cisplatin, FOLFOXIRI, abraxane, an anti-CD40 antibody, oregovomab, Nelfinavir, cetuximab, tegafur, leucovorin and combinations thereof.

In view of the above, the present invention hence also provides a pharmaceutical composition comprising a monoclonal antibody (or antigen-binding fragment or peptide thereof) as described herein or a nucleic acid encoding the same for use in treating a population of patients having been diagnosed with pancreatic ductal adenocarcinoma (PDAC), said PDAC expressing KRAS, HRAS or NRAS wild-type. The antibody may in particular be nimotuzumab.

The pharmaceutical composition is envisaged to comprise a dosage of active agent of between 200 to 400 mg. The term "dosage of (...) between 200 to 400 mg" comprises dosages of, e.g., 200 mg, 250 mg, 300 mg, 350 mg and 400 mg active agent, and dosages in between. The active agent, i.e. the monoclonal antibody (or antigen-binding fragment or peptide thereof), in particular be nimotuzumab.

A better understanding of the present invention and of its advantages will be had from the following examples, offered for illustrative purposes only. The examples are not intended to limit the scope of the present invention in any way.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., "about 20" includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

### EXAMPLES

FOLFIRINOX significantly increases survival in metastatic PC compared to gemcitabine, but its use is limited to selected patients, due its high toxicity. In the majority of cases, gemcitabine (gem) remains the mainstay of palliative treatment, although its modest impact on survival and disease progression. The addition of the EGFR tyrosine kinase inhibitor erlotinib prolonged median survival for only 2 weeks. This study was aimed to investigate the effect of adding Nimotuzumab (nimo), an anti-EGFR monoclonal antibody, to first-line gemcitabine, in pancreatic cancer patients (PCP).

### EXAMPLE 1: Locally advanced or metastatic pancreatic cancer response to gemcitabine plus nimotuzumab

### Treatment

One hundred and ninety two (192) newly diagnosed patients with locally advanced or metastatic pancreatic cancer were randomized and recruited into 2 arms, on the treatment arm patients were administered administered i.v infusion of nimotuzumab at 400 mg dose once a week until progressive disease or unacceptable toxicity and gemcitabine 1000 mg/m² once weekly for 3 weeks, followed by a 1-week rest (d1, 8, 15; q28) and on the control arm patients were administered i.v infusion of placebo once a week until progressive disease or unacceptable toxicity and gemcitabine 1000 mg/m² once weekly for 3 weeks, followed by a 1-week rest (d1, 8, 15; q28).

Tumor response was assessed using CT (Computed Tomography) or MRI (Magnetic Resonance Imaging) and statistically analyzed using RECIST (Response Evaluation Criteria in Solid Tumors), which provides guidelines for identifying complete response, partial response, stable disease, or progressive disease based on tumor size (see, e.g., Therasse et al., February 2000, "New Guidelines to Evaluate the Response to Treatment in Solid Tumors," J. Natl. Cancer Inst. 92(3): 205-216) and overall survival was assessed by the last patient visit, death or loss of follow up.

### EXAMPLE 2: Mutational analysis of KRAS in patients with locally advanced or metastatic pancreatic cancer and relationship with survival.

Out of 192 patients that were randomized into 2 arms, tumor samples were taken from 101 patients (49 from the control arm and 52 from the treatment arm).

To determine if KRAS status correlates with survival the 101 samples were tested. From the KRAS analysis of the 101 samples, 34 samples were KRAS Wild Type (20 from the control arm and 14 from the treatment arm) and 67 samples has the mutation of the KRAS (29 from the control arm and 38 from the treatment arm). Table 1 shows KRAS status of the patients included on the clinical trial by treatment arm.

**Table 1. KRAS status of the locally advanced or metastatic pancreatic cancer patients.**

| **No.** | **Patient ID** | **Treatment Arm** | **KRAS Status** |
|---|---|---|---|
| 1 | 005 | OSAG 101 | Mutation G12D (G35A) |
| 2 | 006 | OSAG 101 | Wildtyp |
| 3 | 007 | Placebo | Mutation G12V (G35T) |
| 4 | 008 | Placebo | Wildtyp |
| 5 | 010 | Placebo | Mutation G12D (G35A) |
| 6 | 011 | OSAG 101 | Wildtyp |
| 7 | 012 | OSAG 101 | Wildtyp |
| 8 | 016 | Placebo | Mutation G12D (G35A) |
| 9 | 018 | OSAG 101 | Mutation G12R (G34C) |
| 10 | 019 | OSAG 101 | Mutation G12D (G35A) |
| 11 | 020 | Placebo | Wildtyp |
| 12 | 021 | Placebo | Wildtyp |
| 13 | 023 | OSAG 101 | Mutation G12D (G35A) |
| 14 | 025 | OSAG 101 | Mutation G12A (G35C) |
| 15 | 026 | Placebo | Wildtyp |
| 16 | 027 | OSAG 101 | Mutation G12D (G35A) |
| 17 | 029 | OSAG 101 | Mutation G12A (G35C) |
| 18 | 032 | Placebo | Mutation G12R (G34C) |
| 19 | 034 | Placebo | Mutation G12D (G35A) |
| 20 | 036 | OSAG 101 | Wildtyp |
| 21 | 038 | Placebo | Mutation G12R (G34C) |
| 22 | 040 | OSAG 101 | Mutation G12D (G35A) |
| 23 | 041 | OSAG 101 | Mutation G12R (G34C) |
| 24 | 043 | Placebo | Wildtyp |
| 25 | 045 | Placebo | Wildtyp |
| 26 | 050 | OSAG 101 | Wildtyp |
| 27 | 051 | Placebo | Mutation G12V (G35T) |
| 28 | 052 | Placebo | Mutation G12D (G35A) |
| 29 | 053 | OSAG 101 | Mutation G12R (G34C) |
| 30 | 054 | OSAG 101 | Mutation G12V (G35T) |
| 31 | 056 | Placebo | Mutation G12S (G34A) |
| 32 | 057 | OSAG 101 | Mutation G12D (G35A) |
| 33 | 058 | OSAG 101 | Mutation G12V (G35T) |
| 34 | 059 | Placebo | Mutation G12A (G35C) |
| 35 | 061 | Placebo | Mutation G12D (G35A) |
| 36 | 062 | OSAG 101 | Mutation G12D (G35A) |
| 37 | 064 | OSAG 101 | Wildtyp |
| 38 | 066 | OSAG 101 | Mutation G12D (G35A) |
| 39 | 067 | Placebo | Wildtyp |
| 40 | 068 | Placebo | Mutation G12D (G35A) |
| 41 | 069 | OSAG 101 | Wildtyp |
| 42 | 070 | OSAG 101 | Mutation G12V (G35T) |
| 43 | 072 | Placebo | Wildtyp |
| 44 | 073 | OSAG 101 | Mutation G12D (G35A) |
| 45 | 074 | OSAG 101 | Mutation G12D (G35A) |
| 46 | 076 | OSAG 101 | Mutation G12D (G35A) |
| 47 | 077 | OSAG 101 | Mutation G12D (G35A) |
| 48 | 078 | Placebo | Mutation G12D (G35A) |
| 49 | 080 | Placebo | Mutation G12V (G35T) |
| 50 | 082 | Placebo | Mutation G12D (G35A) |
| 51 | 083 | OSAG 101 | Wildtyp |
| 52 | 084 | Placebo | Mutation G12V (G35T) |
| 53 | 085 | Placebo | Wildtyp |
| 54 | 086 | Placebo | Wildtyp |
| 55 | 087 | OSAG 101 | Mutation G12D (G35A) |
| 56 | 088 | OSAG 101 | Mutation G12V (G35T) |
| 57 | 089 | OSAG 101 | Mutation G12D (G35A) |
| 58 | 090 | Placebo | Mutation G12V (G35T) |
| 59 | 091 | Placebo | Mutation G12V (G35T) |
| 60 | 092 | OSAG 101 | Wildtyp |
| 61 | 093 | OSAG 101 | Mutation G12D (G35A) |
| 62 | 095 | OSAG 101 | Mutation G12D (G35A) |
| 63 | 096 | Placebo | Mutation G12D (G35A) |
| 64 | 097 | Placebo | Wildtyp |
| 65 | 098 | Placebo | Mutation G12D (G35A) |
| 66 | 099 | OSAG 101 | Mutation G12V (G35T) |
| 67 | 100 | OSAG 101 | Wildtyp |
| 68 | 101 | OSAG 101 | Mutation G12V (G35T) |
| 69 | 102 | Placebo | Wildtyp |
| 70 | 103 | OSAG 101 | Mutation G12R (G34C) |
| 71 | 104 | Placebo | Mutation G12D (G35A) |
| 72 | 105 | OSAG 101 | Wildtyp |
| 73 | 106 | Placebo | Mutation G12V (G35T) |
| 74 | 109 | OSAG 101 | Mutation G12V (G35T) |
| 75 | 110 | Placebo | Wildtyp |
| 76 | 112 | Placebo | Mutation G12D (G35A) |
| 77 | 114 | Placebo | Wildtyp |
| 78 | 118 | OSAG 101 | Mutation G12V (G35T) |
| 79 | 119 | Placebo | Wildtyp |
| 80 | 121 | Placebo | Mutation G12V (G35T) |
| 81 | 124 | OSAG 101 | Mutation G12V (G35T) |
| 82 | 125 | OSAG 101 | Wildtyp |
| 83 | 129 | OSAG 101 | Mutation G12V (G35T) |
| 84 | 133 | OSAG 101 | Mutation G12D (G35A) |
| 85 | 134 | OSAG 101 | Wildtyp |
| 86 | 135 | Placebo | Mutation G12V (G35T) |
| 87 | 140 | OSAG 101 | Mutation G12D (G35A) |
| 88 | 143 | OSAG 101 | Wildtyp |
| 89 | 145 | Placebo | Mutation G12V (G35T) |
| 90 | 149 | OSAG 101 | Mutation G12D (G35A) |
| 91 | 150 | Placebo | Mutation G12D (G35A) |
| 92 | 158 | OSAG 101 | Mutation G12D (G35A) |
| 93 | 161 | Placebo | Wildtyp |
| 94 | 164 | OSAG 101 | Mutation G12D (G35A) |
| 95 | 165 | Placebo | Wildtyp |
| 96 | 168 | OSAG 101 | Mutation G12D (G35A) |
| 97 | 169 | Placebo | Wildtyp |
| 98 | 171 | Placebo | Wildtyp |
| 99 | 174 | Placebo | Mutation G12D (G35A) |
| 100 | 179 | Placebo | Wildtyp |
| 101 | 189 | Placebo | Mutation G12V (G35T) |

The Kaplan-Meier curves of the locally advanced or metastatic pancreatic cancer patient with KRAS mutated from the placebo-gemcitabine arm compared to the patients from the nimotuzumab-gemcitabine arm shows no difference in term of progression-free-survival (PFS) and overall survival (OS) **(****FIGURE 3****)**

The Kaplan-Meier curves of the locally advanced or metastatic pancreatic cancer patient with KRAS wild type from the placebo-gemcitabine arm compared to the patients from the nimotuzumab-gemcitabine arm shows a significant difference in favour of the treatment arm in term of progression-free-survival (PFS) and overall survival (OS) **(****FIGURE 4****)**

### Safety

There were a total of 212 serious adverse events (SAEs) reported in 115 patients, 104 SAEs from the placebo-gemcitabine control arm reported in 61/93 (65.6%) patients and 108 SAEs from nimotuzumab plus gemcitabine arm reported in 54/93 (58.1%) patients. Of the 108 SAEs, reported on the nimotuzumab arm, 26 SAEs in 15/93 (16.1%) patients were reported to be related to the study drug. Most common SAEs reported to be related to study drug were general physical health deterioration (3 subjects, 3.23 %), nausea (2 subjects, 2.15%), vomiting (2 subjects, 2.15%), and thrombocytopenia (2 subjects, 2.15%).

### Results

As a primary endpoint, overall survival (OS) in the intention-to-treat (ITT) population was evaluated. The OS was assessed by the date of the last visit, death or loss of the follow up. The OS was initially calculated for all population and then a subset analysis was run taking into consideration the KRAS status of each of the patients on each treatment arm. Secondary endpoints included progression free survival (PFS), assessed by the day of the progression or the death, safety, assessed by the incidence of adverse events (AE) or serious adverse events (SAE), objective response rate (ORR), assessed by the number of patients on each treatment arm with Complete Response (CR) and Partial Response (PR) to the treatment ((CR + PR) / N, No. patients in the arm)) and Quality of Life (QoL), assessed using EORTC quality of life questionnaire QLQ-C30 version 2.0 (EORTC, NL).

Progression-free-survival (PFS) is defined as the length of time during and after the treatment of a disease, such as cancer, that a patient lives with the disease without worsening of the symptoms and was measured from date of randomization to the occurrence of progression or death or last date of observation without progression.

Complete response is defined as the disappearance of all target cancer lesions and was measured at weeks 8, 16, and 24

Partial Response (PR) is defined as at least a 30% decrease in the sum of the longest dimensions of target lesions, taking as a reference the baseline sum of longest dimensions and measured at weeks 8, 16 and 24

Quality-of-Life was assessed with the EORTC QLQ - C30 module as reviewed in Fayers et al. Eur J Cancer. 2002 Mar;38 Suppl 4:S125-33 and was measured at the following time points: (1) Baseline within 7 days prior to first study medication administration on Day 1, (2) In week 6, 14 and then every 8 weeks, and (3) At the end of treatment.

In the analysis of subgroup of KRAS WT, overall survival was significantly improved on the patients with locally advanced or metastatic pancreatic cancer that received the treatment of nimotuzumab and gemcitabine with an increase of median overall survival in 6 months when it was compared to the control arm that received placebo-gemcitabine. (see **TABLE 2).**

## Claims

1. Nimotuzumab for use in a method of treating pancreatic ductal adenocarcinoma (PDAC) in a patient having PDAC, wherein the PDAC expresses KRAS wild-type.

2. Nimotuzumab for the use of claim 1, wherein the wild-type KRAS is **characterized in** having a sequence corresponding to SEQ ID NO.: 7 or SEQ ID No. 24.

3. Nimotuzumab for the use of claim 1 or claim 2, wherein the wild-type KRAS is **characterized in that** is capable of turning off the signaling pathway by catalyzing hydrolysis of guanosine triphosphates (GTP) to guanosine diphosphates.

4. Nimotuzumab for the use of claim 3, wherein the wild-type KRAS does not comprise a mutation in any of amino acid residues of codon G12, G13, Q61, A146 or D154.

5. Nimotuzumab for the use of claim 4, wherein the lacking mutation of KRAS is selected from the group consisting of G12D, G12A, G12R, G12C, G12S, G12V and G13D.

6. Nimotuzumab for the use of any of the preceding claims, wherein the patient is selected by determining the presence or absence of mutant and/or wild-type KRAS in a PDAC tumor sample of the patient, wherein patients in whose tumor samples mutant KRAS is detected and/or wild-type KRAS is not detected are not selected for treatment, whereas patients in whose wild-type KRAS is detected and/or mutant KRAS is not detected are selected for treatment,
wherein determining the expression of wild-type KRAS in a PDAC tumor sample preferably comprises amplifying a KRAS nucleic acid from the tumor and sequencing the nucleic acid and/or comprises detecting mutant and/or wild-type KRAS polypeptide in a sample of the PDAC tumor.

7. Nimotuzumab for the use according to any of the preceding claims , wherein the use further comprises the co-administration of an anti-cancer agent,
wherein the anti-cancer agent is preferably selected from the group consisting of a chemotherapeutic agent, an anti-neoplastic agent and a biological molecule,
wherein the anti-cancer agent is preferably co-administered antecedently, simultaneously and/or subsequently to administration of the monoclonal antibody, antigen binding fragment or peptide thereof, or
wherein the anti-cancer agent is preferably selected from gemtabicine, FOLFIRINOX, erlotinib, 5-fluorouracil, paclitaxel, nab-paclitaxel, docetaxel, capecitabine, oxaliplatin cisplatin, FOLFOXIRI, abraxane, an anti-CD40 antibody, oregovomab, Nelfinavir, cetuximab, tegafur, leucovorin.

8. Nimotuzumab for the use of claim 7, wherein the co-administered anti-cancer agent is gemcitabine.

9. Nimotuzumab for the use according to any of the preceding claims, wherein the patients have been diagnosed with locally advanced and/or metastatic pancreatic ductal adenocarcinoma (PDAC).

10. Nimotuzumab for the use according to any of the preceding claims, wherein the patients are treated with nimotuzumab in a dosage range of between 200 and 400 mg once a week, wherein the weakly treatment with the dosage of nimotuzumab of between 200 and 400 mg is preferably carried out over a period of time of 12, 24 or 36 weeks, wherein after the weekly treatment for 12, 24 or 36 weeks, the patient is preferably treated with a dosage of nimotuzumab of between 200 and 400 mg once every 3 weeks.

11. A method of predicting whether a patient suffering from pancreatic ductal adenocarcinoma (PDAC) will be nonresponsive to treatment with nimotuzumab, comprising determining the presence or absence of a KRAS mutation in a PDAC tumor in the patient; wherein the presence of a KRAS, mutation in a sample of the tumor indicates that the patient will be nonresponsive to treatment with said antibody, antibody fragment or peptide thereof,
wherein the KRAS mutation is preferably in the codon of one or more of amino acid residues G12, G13, Q61, A146, or D154 of SEQ ID NO.7 or SEQ ID No. 24.

12. The method of claim 11, wherein determining the presence or absence of a KRAS mutation in a PDAC tumor comprises amplifying a KRAS nucleic acid from the tumor and sequencing the nucleic acid and/or comprises detecting mutant KRAS polypeptide in a sample of the tumor.

## Patentansprüche

1. Nimotuzumab zur Verwendung in einem Verfahren zur Behandlung des duktalen Pankreasadenokarzinoms (PDAC) bei einem Patienten mit PDAC, wobei das PDAC das Wildtyp-KRAS exprimiert.

2. Nimotuzumab zur Verwendung nach Anspruch 1, wobei das Wildtyp-KRAS **dadurch gekennzeichnet ist, dass** es eine Sequenz, entsprechend SEQ ID Nr.: 7 oder SEQ ID Nr.: 24, hat.

3. Nimotuzumab zur Verwendung nach Anspruch 1 oder Anspruch 2, wobei das Wildtyp-KRAS **dadurch gekennzeichnet ist, dass** es in der Lage ist, den Signalweg durch Katalysieren der Hydrolyse von Guanosintriphosphaten (GTP) zu Guanosindiphosphaten auszuschalten.

4. Nimotuzumab zur Verwendung nach Anspruch 3, wobei das Wildtyp-KRAS keine Mutation in einem der Aminosäurereste von Codon G12, G13, Q61, A146 oder D154 umfasst.

5. Nimotuzumab zur Verwendung nach Anspruch 4, wobei die fehlende Mutation von KRAS ausgewählt ist aus der Gruppe, bestehend aus G12D, G12A, G12R, G12C, G12S, G12V und G13D.

6. Nimotuzumab zur Verwendung nach einem der vorhergehenden Ansprüche, wobei der Patient ausgewählt wird durch Bestimmen des Vorhandenseins oder des Nichtvorhandenseins eines mutierten oder Wildtyp-KRAS in einer PDAC-Tumorprobe des Patienten, wobei die Patienten, in deren Tumorproben mutiertes KRAS festgestellt wurde und/oder Wildtyp-KRAS nicht festgestellt wurde, nicht für eine Behandlung ausgewählt werden, während Patienten, bei denen Wildtyp-KRAS festgestellt wird und/oder mutiertes KRAS nicht festgestellt wird, für die Behandlung ausgewählt werden,
wobei die Bestimmung der Expression von Wildtyp-KRAS in einer PDAC-Tumorprobe vorzugweise das Amplifizieren einer KRAS-Nukleinsäure aus dem Tumor und das Sequenzieren der Nukleinsäure und/oder das Feststellen eines mutierten und/oder Wildtyp-KRAS-Polypeptids in einer Probe des PDAC-Tumors umfasst

7. Nimotuzumab zur Verwendung nach einem der vorhergehenden Ansprüche, wobei die Verwendung weiterhin die Co-Verabreichung eines Antikrebsmittels umfasst,
wobei das Antikrebsmittel vorzugsweise ausgewählt ist aus der Gruppe, bestehend aus einem Chemotherapeutikum, einem antineoplastischen Mittel und einem biologischen Molekül,
wobei das Antikrebsmittel vorzugsweise vor, gleichzeitig mit und/oder nach der Verabreichung des monoklonalen Antikörpers, des antigenbindenden Fragments oder des Peptids davon co-verabreicht wird, oder
wobei das Antikrebsmittel vorzugsweise ausgewählt ist aus Gemtabicin, FOLFIRINOX, Erlotinib, 5-Fluoruracil, Paclitaxel, nab-Paclitaxel, Docetaxel, Capecitabin, Oxaliplatin, Cisplatin, FOLFOXIRI, Abraxan, einem Anti-CD40-Antikörper, Oregovomab, Nelfinavir, Cetuximab, Tegafur, Leucovorin.

8. Nimotuzumab zur Verwendung nach Anspruch 7, wobei das coverabreichte Antikrebsmittel Gemcitabin ist.

9. Nimotuzumab zur Verwendung nach einem der vorhergehenden Ansprüche, wobei die Patienten mit einem lokal fortgeschrittenen und/oder metastasierendem duktalen Pankreasadenokarzinom (PDAC) diagnostiziert wurden.

10. Nimotuzumab zur Verwendung nach einem der vorhergehenden Ansprüche, wobei die Patienten mit Nimotuzumab in einem Dosierungsbereich von zwischen 200 und 400 mg ein Mal pro Woche behandelt werden, wobei die wöchentliche Behandlung mit der Dosierung von Nimotuzumab von zwischen 200 und 400 mg vorzugsweise über einen Zeitraum von 12, 24 oder 36 Wochen durchgeführt wird, wobei der Patient nach der wöchentlichen Behandlung für 12, 24 oder 36 Wochen vorzugsweise mit einer Dosierung von Nimotuzumab von zwischen 200 und 400 mg ein Mal alle 3 Wochen behandelt wird.

11. Verfahren zur Vorhersage, ob ein Patient mit duktalem Pankreasadenokarzinom (PDAC) auf eine Behandlung mit Nimotuzumab nicht anspricht, umfassend das Bestimmen des Vorhandenseins oder Nichtvorhandenseins einer KRAS-Mutation in einem PDAC-Tumor bei dem Patienten, wobei das Vorhandensein einer KRAS-Mutation in einer Tumorprobe anzeigt, dass der Patient auf die Behandlung mit dem Antikörper, dem Antikörperfragment oder einem Peptid davon nicht ansprechen wird,
wobei die KRAS-Mutation vorzugsweise in dem Codon von einem oder mehreren der Aminosäurereste G12, G13, Q61, A146, oder D154 von SEQ ID Nr.: 7 oder SEQ ID Nr.: 24 vorhanden ist.

12. Verfahren nach Anspruch 11, wobei das Bestimmen des Vorhandenseins oder Nichtvorhandenseins einer KRAS-Mutation in einem PDAC-Tumor Folgendes das Amplifizieren einer KRAS-Nukleinsäure aus dem Tumor und das Sequenzieren der Nukleinsäure und/oder das Feststellen eines mutierten KRAS-Polypeptids in einer Probe des Tumors umfasst.

## Revendications

1. Nimotuzumab à utiliser dans un procédé de traitement du adénocarcinome canalaire du pancréas (PDAC) chez un patient ayant le PDAC, dans lequel le PDAC exprime le KRAS de type sauvage.

2. Nimotuzumab à utiliser selon la revendication 1, dans lequel le KRAS de type sauvage est caractérisé en ayant une séquence correspondant à SEQ ID N°: 7 ou SEQ ID N°: 24.

3. Nimotuzumab à utiliser selon la revendication 1 ou la revendication 2, dans lequel le KRAS de type sauvage est **caractérisé en ce qu'**il est capable de désactiver la voie de signalisation en catalysant l'hydrolyse des triphosphates de guanosine (GTP) aux diphosphates de guanosine.

4. Nimotuzumab à utiliser selon la revendication 3, dans lequel le KRAS de type sauvage ne comprend pas une mutation dans l'un des résidus d'acides aminés du codon G12, G13, Q61, A146 ou D154.

5. Nimotuzumab à utiliser selon la revendication 4, dans lequel la mutation manquante de KRAS est choisie parmi le groupe, constitué de G12D, G12A, G12R, G12C, G12S, G12V et G13D.

6. Nimotuzumab à utiliser selon l'une quelconque des revendications précédentes, dans lequel le patient est choisi en déterminant la présence ou l'absence de KRAS mutant et/ou de type sauvage dans un échantillon du tumeur PDAC du patient, dans lequel des patients dans les échantillons du tumeur desquels le KRAS mutant est détecté et/ou le KRAS de type sauvage n'est pas détecté, ne sont pas choisis pour le traitement, tandis que des patients, où le KRAS de type sauvage est détecté et/ou le KRAS mutant n'est pas détecté, sont choisis pour le traitement,
dans lequel la détermination de l'expression du KRAS de type sauvage dans un échantillon du tumeur PDAC comprend, de préférence, l'amplification d'un acide nucléique KRAS du tumeur, et le séquençage de l'acide nucléique et/ou comprend la détection du polypeptide de KRAS mutant et/ou de KRAS de type sauvage dans un échantillon du tumeur PDAC.

7. Nimotuzumab à utiliser selon l'une quelconque des revendications précédentes, dans lequel l'utilisation comprend en outre la co-administration d'un agent anticancéreux,
dans lequel l'agent anticancéreux est, de préférence, choisi parmi le groupe constitué d'un agent chimiothérapeutique, d'un agent anti-néoplastique et d'une molécule biologique,
dans lequel l'agent anticancéreux est, de préférence, co-administré avant, simultanément avec et/ou après l'administration de l'anticorps monoclonal, du fragment de liaison d'antigène ou du peptide de celui-ci, ou
dans lequel l'agent anticancéreux est, de préférence, choisi parmi gemtabicine, FOLFIRINOX, erlotinibe, 5-fluorouracile, paclitaxel, nab-paclitaxel, docétaxel, capécitabine, oxaliplatine, cisplatine, FOLFOXIRI, abraxane, un anticorps anti-CD40, orégovomab, nelfinavir, cetuximab, tégafur, leucovorine.

8. Nimotuzumab à utiliser selon la revendication 7, dans lequel l'agent anticancéreux co-administré est la gemcitabine.

9. Nimotuzumab à utiliser selon l'une quelconque des revendications précédentes, dans lequel les patients ont été diagnostiqués avec l'adénocarcinome canalaire du pancréas (PDAC) localement avancé et/ou métastatique.

10. Nimotuzumab à utiliser selon l'une quelconque des revendications précédentes, dans lequel les patients sont traités avec nimotuzumab dans une plage de dosage comprise entre 200 et 400 mg une fois par semaine, dans lequel le traitement hebdomadaire avec le dosage de nimotuzumab compris entre 200 et 400 mg est, de préférence, réalisé sur une période de temps de 12, 24 ou 36 semaines, dans lequel, après le traitement hebdomadaire pour 12, 24 ou 36 semaines, le patient est, de préférence, traité avec un dosage de nimotuzumab compris entre 200 et 400 mg une fois toutes les 3 semaines.

11. Procédé de prédiction, si un patient souffrant de l'adénocarcinome canalaire du pancréas (PDAC) ne réagira pas au traitement avec nimotuzumab, comportant la détermination de la présence ou de l'absence d'une mutation de KRAS dans un tumeur PDAC chez le patient, dans lequel la présence d'une mutation de KRAS dans un échantillon du tumeur indique que le patient ne réagira pas au traitement avec ledit anticorps, le fragment d'anticorps ou le peptide de celui-ci,
dans lequel la mutation de KRAS est, de préférence, dans le codon de l'un ou plusieurs de résidus d'acides aminés G12, G13, Q61, A146, ou D154 de SEQ ID N° 7 ou SEQ ID N° 24.

12. Procédé selon la revendication 11, dans lequel la détermination de la présence ou de l'absence d'une mutation de KRAS dans un tumeur PDAC comporte l'amplification d'un acide nucléique KRAS du tumeur et le séquençage de l'acide nucléique, et/ou comporte la détection du polypeptide du KRAS mutant dans un échantillon du tumeur.
